# EUROPEAN PATENT APPLICATION

(11) **EP 4 167 173 A1**
(43) Date of publication of application: **19.04.2023**
(21) Application number: 20939988.0
(22) Date of filing: 11.06.2020
(51) Int. Cl.: G06Q 50/10, G16H 10/00

(54) **INFORMATION SYSTEM, INFORMATION TERMINAL, IMMUNITY CERTIFICATE MANAGEMENT SYSTEM, INFORMATION PROCESSING METHOD, AND NON-TRANSITORY COMPUTER-READABLE MEDIUM**

(71) Applicant: NEC Corporation, 108-8001 Tokyo (JP)
(72) Inventor: KOIDE Toshio, Tokyo 108-8001 (JP)
(74) Representative: Betten & Resch
(86) International application number: PCT/JP2020/023054
(87) International publication number: WO 2021/250857

(57) **Abstract**

An object of the present disclosure is to provide an information system, an information terminal, an immunity certificate management system, an information processing method, and a non-transitory computer readable medium storing a program for effectively using immunity certificates. An information system (30) according to an aspect of the present disclosure includes storage means (31) for storing an immunity certificate, update information storage means (32) for storing, when the immunity certificate stored in the storage means (31) is updated, update information of the immunity certificate as a hash value related to the immunity certificate, and output means (33) for outputting the update information stored in the update information storage means (32).

## Description

### Technical Field

The present disclosure relates to an information system, an information terminal, an immunity certificate management system, an information processing method, and a non-transitory computer readable medium storing a program.

### Background Art

As advances in information and communication technology have been made, a movement to effectively use medical information online has grown.

For example, Patent Literature 1 discloses that a plurality of medical institutions form a block-chaining system to manage patient information.

### Citation List

### Patent Literature

Patent Literature 1: Japanese Unexamined Patent Application Publication No. 2020-052457

### Summary of Invention

### Technical Problem

As of June 2020, various efforts to control the spread of the infectious disease caused by the new type of coronavirus (COVID-19) have been made in Japan and in other countries around the world.

Local municipal governments and national governments have asked or ordered all the residents to refrain from doing nonessential activities, or have imposed lockdowns in order to curb the spread of the virus. However, these methods make even people who are considered less susceptible to the virus suspend social activities, and hence may unduly suppress economic activities.

In view of this fact, it has been proposed, as a way to achieve both infection control and avoid suppression of economic activities at the same time, to enable individuals who are at low risk of the infectious disease to prove the risk is low by using immunity certificates. The fact "being at low risk of the infection disease" means, for example, that an individual has acquired immunity to the virus, that an individual is unlikely to be infected, that even if an individual is infected, the effect of the disease caused thereby is mild rather than severe, or that the risk of infecting others is low.

For example, by effectively using immunity certificates by which it is possible to determine the risk of an individual of infection of the infectious diseases, a company to which an individual commutes or a facility to which an individual visits determines the risk of the infection of the individual and permits the individual to commute to the company or enter the facility. In this way, it is possible to minimize the number of people whose social activities should be restricted, and thereby to enable people who are less susceptible to the virus to resume economic activities as much as possible.

An object of the present disclosure is to provide an information system, an information terminal, an immunity certificate management system, an information processing method, and a non-transitory computer readable medium storing a program for effectively using immunity certificates.

### Solution to Problem

An information system according to an aspect of an example embodiment includes: storage means for storing an immunity certificate; update information storage means for storing, when the immunity certificate stored in the storage means is updated, update information of the immunity certificate as a hash value related to the immunity certificate; and output means for outputting the update information stored in the update information storage means.

An information system according to another aspect of an example embodiment includes: storage means for storing an immunity certificate in a format for secret calculation; secret calculation means for performing the secret calculation on the immunity certificate stored in the storage means; and output means for outputting a result of the secret calculation performed by the secret calculation means.

An information terminal according to an aspect of an example embodiment includes: acquisition means for acquiring immunity certificate information; sharing means for secretly sharing the immunity certificate information acquired by the acquisition means into a plurality of information pieces; and transmitting means for transmitting the plurality of information pieces generated by the sharing means to another apparatus.

An information terminal according to another aspect of an example embodiment includes: acquisition means for acquiring information based on an immunity certificate of an individual; receiving means for receiving a hash value from another apparatus as information indicating genuineness of the immunity certificate; and determination means for determining, based on the hash value acquired by the receiving means, reliability of the information based on the immunity certificate.

An immunity certificate management system according to an aspect of an example embodiment includes: an information system; and an information terminal configured to communicate with the information system, in which the information system includes: storage means for storing an immunity certificate; update information storage means for storing, when the immunity certificate stored in the storage means is updated, update information of the immunity certificate as a hash value related to the immunity certificate; and transmitting means for transmitting the update information stored in the update information storage means to the information terminal, and the information terminal includes receiving means for receiving the transmitted update information.

An immunity certificate management system according to another aspect of an example embodiment includes: an information system; and an information terminal configured to communicate with the information system, in which the information system includes: storage means for storing an immunity certificate in a format for secret calculation; secret calculation means for performing the secret calculation on the immunity certificate stored in the storage means; and transmitting means for transmitting a result of the secret calculation performed by the secret calculation means to the information terminal, and the information terminal includes receiving means for receiving the result of the secret calculation.

An information processing method according to a first aspect in accordance with an example embodiment includes: a step of storing an immunity certificate; a step of storing, when the stored immunity certificate is updated, update information of the immunity certificate as a hash value related to the immunity certificate; and a step of outputting the stored update information.

An information processing method according to a second aspect in accordance with an example embodiment includes: a step of storing an immunity certificate in a format for secret calculation; a step of performing the secret calculation on the stored immunity certificate; and a step of outputting a result of the secret calculation.

An information processing method according to a third aspect in accordance with an example embodiment includes: a step of acquiring immunity certificate information; a step of secretly sharing the acquired immunity certificate information into a plurality of information pieces; and a step of transmitting the secrecy-shared plurality of information pieces to another apparatus.

An information processing method according to a fourth aspect in accordance with an example embodiment includes: a step of acquiring information based on an immunity certificate of an individual; a step of communicating with another apparatus and thereby acquiring a hash value from the other apparatus as information indicating genuineness of the immunity certificate; and a step of determining, based on the acquired hash value, reliability of the information based on the immunity certificate.

A non-transitory computer readable medium according to a first aspect in accordance with an example embodiment stores a program for causing a computer to perform: a step of storing an immunity certificate; a step of storing, when the stored immunity certificate is updated, update information of the immunity certificate as a hash value related to the immunity certificate; and a step of outputting the stored update information.

A non-transitory computer readable medium according to a second aspect in accordance with an example embodiment stores a program for causing a computer to perform: a step of storing an immunity certificate in a format for secret calculation; a step of performing the secret calculation on the stored immunity certificate; and a step of outputting a result of the secret calculation.

A non-transitory computer readable medium according to a third aspect in accordance with an example embodiment stores a program for causing a computer to perform: a step of acquiring immunity certificate information; a step of secretly sharing the acquired immunity certificate information into a plurality of information pieces; and a step of transmitting the secrecy-shared plurality of information pieces to another apparatus.

A non-transitory computer readable medium according to a fourth aspect in accordance with an example embodiment stores a program for causing a computer to perform: a step of acquiring information based on an immunity certificate of an individual; a step of communicating with another apparatus and thereby acquiring a hash value from the other apparatus as information indicating genuineness of the immunity certificate; and a step of determining, based on the acquired hash value, reliability of the information based on the immunity certificate.

### Advantageous Effects of Invention

According to the present disclosure, it is possible to provide an information system, an information terminal, an immunity certificate management system, an information processing method, and a non-transitory computer readable medium storing a program for effectively using immunity certificates.

### Brief Description of Drawings

Fig. 1 is a schematic diagram of an immunity certificate management system according to a first example embodiment;
Fig. 2 is a block diagram of an information terminal 10 according to the first example embodiment;
Fig. 3 is a block diagram of an information terminal 20 according to the first example embodiment;
Fig. 4 is a block diagram of an information system 30 according to the first example embodiment;
Fig. 5 is a block diagram of an information system 40 according to a second example embodiment;
Fig. 6 is a schematic diagram of an immunity passport system according to a third example embodiment;
Fig. 7 is a block diagram showing a configuration of a personal terminal 200 according to the third example embodiment;
Fig. 8 is an example of displayed infectious-disease risk level information according to the third example embodiment;
Fig. 9 is a block diagram showing a configuration of a medical institution terminal 300 according to the third example embodiment;
Fig. 10 is a block diagram showing a configuration of an administrative institution terminal 400 according to the third example embodiment;
Fig. 11 is a block diagram showing a configuration of a business proprietor terminal 500 according to the third example embodiment;
Fig. 12 is a block diagram showing a configuration of an immune passport base system 600 according to the third example embodiment;
Fig. 13 is an image of processing performed by an update unit 603 according to the third example embodiment;
Fig. 14 is a flowchart showing processes performed by a personal terminal 200 according to the third example embodiment;
Fig. 15 is a flowchart showing processes performed by a business proprietor terminal 500 according to the third example embodiment;
Fig. 16 is a flowchart showing processes performed by the immune passport base system 600 according to the third example embodiment;
Fig. 17 is a block diagram showing a configuration of a personal terminal 210 according to a sixth example embodiment;
Fig. 18 is a block diagram showing a configuration of a business proprietor terminal 510 according to a ninth example embodiment; and
Fig. 19 is a block diagram showing an example of a hardware configuration of a terminal or a system.

### Example Embodiment

### First Example Embodiment

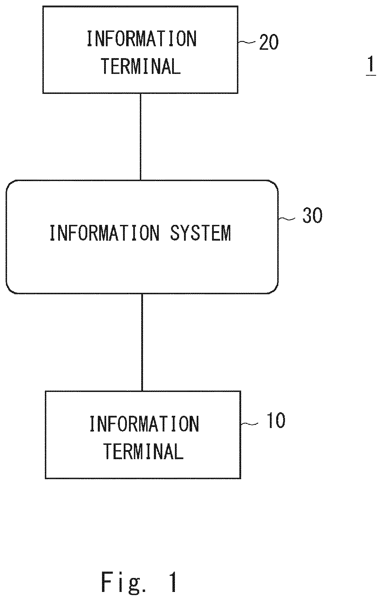

An example embodiment according to the present disclosure will be described hereinafter with reference to the drawings. Fig. 1 is a schematic diagram of an immunity certificate management system according to a first example embodiment. The immunity certificate management system 1 includes information terminals 10 and 20, and an information system 30. The immunity certificate management system 1 is a system for enabling immunity certificates of individuals or information related thereto to be effectively used.

Note that the "immunity certificate" refers to a set of data with which it is possible to estimate a risk of an individual for infection or development of a certain infection or disease. Although its details will be described in a third example embodiment, specific examples of the immunity certificate include biometric information related to the physical condition of an individual, a medical history thereof, a movement history thereof, and the like.

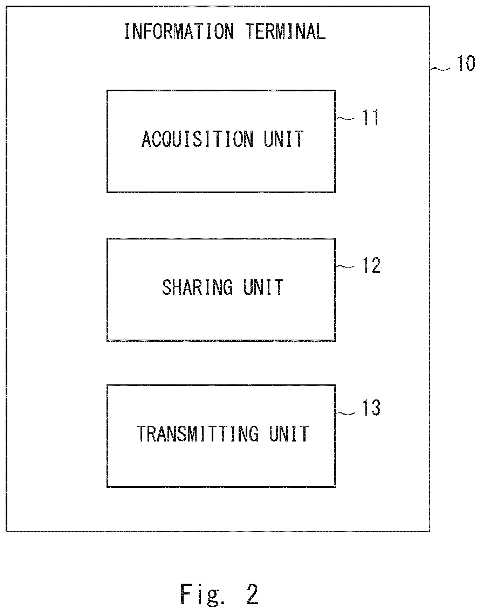

Fig. 2 is a block diagram of the information terminal 10. The information terminal 10 is a terminal used by an individual, and examples thereof include a computer and a portable (or mobile) information terminal (e.g., a smartphone). The information terminal 10 includes an acquisition unit 11, a sharing unit 12, and a transmitting unit 13.

The acquisition unit 11 acquires immunity certificate information of an individual. The "immunity certificate information of an individual" is, for example, information such as biometric information, a medical history, and a movement history over a predetermined period. This immunity certificate information itself may be used as an immunity certificate, or an immunity certificate may be formed by combining a plurality of pieces of immunity certificate information. Examples of the method for acquiring immunity certificate information include entering information through an operation unit, photographing, and acquiring online.

The sharing unit 12 secretly shares (i.e., secretly divides) the immunity certificate information acquired by the acquisition unit 11 into a plurality of information pieces. In this way, even if one information piece is leaked, the immunity certificate information cannot be reproduced and the individual cannot be identified.

The transmitting unit 13 transmits the plurality of information pieces generated by the sharing unit 12 to the information system 30, i.e., to another apparatus. As will be described later, the information system 30 can perform secret calculation by using the transmitted information.

Since the information terminal 10 transmits the immunity certificate information, which is secretly shared (i.e., secretly divided) into a plurality of information pieces, it is possible to effectively use the immunity certificate as well as protecting the privacy of the individual.

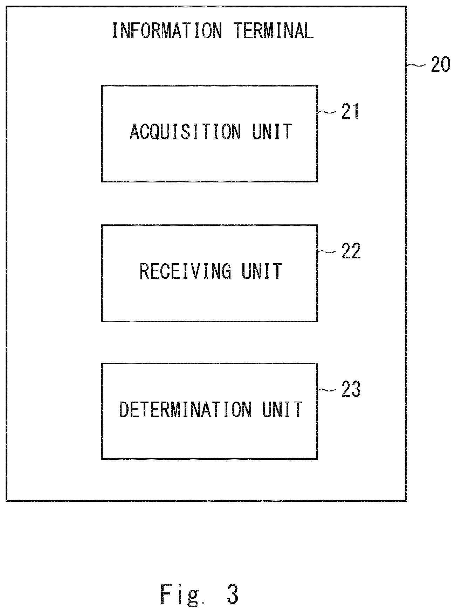

Fig. 3 is a block diagram of the information terminal 20. The information terminal 20 is, for example, a terminal used by a business proprietor, and examples thereof include a computer and a portable (or mobile) information terminal (e.g., a smartphone). The information terminal 20 includes an acquisition unit 21, a receiving unit 22 and a determination unit 23.

The acquisition unit 21 acquires information based on an immunity certificate of an individual. The "information based on an immunity certificate of an individual" is not the immunity certificate itself, but is a result of processing performed based on the immunity certificate and can be effectively used by a person other than the individual (e.g., a business proprietor). Examples of the information based on an immunity certificate include infectious-disease risk level information indicating a risk of being infected by an infectious disease. As will be described later, for example, the acquisition unit 21 can receive information based on an immunity certificate from the information terminal 10. However, the acquisition unit 21 may receive the information from the information system 30 or other apparatuses, or may acquire the information through photographing or the like.

The receiving unit 22 receives a hash value from the information system 30, i.e., from the other apparatus, as information indicating genuineness of the immunity certificate. Note that the receiving unit 22 can also receive a hash value from an apparatus or the like other than the information system 30.

The determination unit 23 determines, based on the hash value acquired by the receiving unit 22, the reliability of the information based on the immunity certificate acquired by the acquisition unit 21. As described above, the "information based on an immunity certificate of an individual" refers to a result of processing performed based on an immunity certificate. Therefore, if the immunity certificate, on which the calculation is based, is incorrect, it means that the information based on an immunity certificate of the individual is unreliable.

When the hash value is genuine, the determination unit 23 determines that the immunity certificate acquired by the acquisition unit 21 is reliable. On the other hand, when the hash value is incorrect, the determination unit 23 determines that the immunity certificate acquired by the acquisition unit 21 is unreliable.

Since the information terminal 20 determines the reliability of the information based on the immunity certificate by using the information indicating the genuineness of the immunity certificate, the user (e.g., a business proprietor) of the information terminal 20 can, after confirming that the immunity certificate is reliable, effectively use the information based on the immunity certificate.

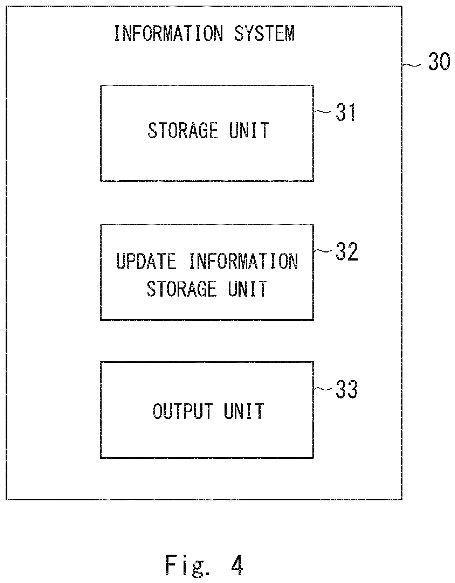

Fig. 4 is a block diagram of the information system 30. The information system 30 is operated by, for example, a third party different from the users of the information terminals 10 and 20. The information system 30 is a system including one or a plurality of computers (e.g., a group of servers), and includes a storage unit 31, an update information storage unit 32, and an output unit 33.

The storage unit 31 stores an immunity certificate of one person or a plurality of immunity certificates of a plurality of persons. Regarding the storage, an immunity certificate may be stored in such a place or such a format that the stored immunity certificate cannot be acquired from an external terminal, but the storage method is not limited to such methods.

When the immunity certificate stored in the storage unit 31 is updated, the update information storage unit 32 stores update information of the updated immunity certificate as a hash value related to that immunity certificate. That is, the hash value indicates whether the immunity certificate is genuine or not.

The "hash value related to an immunity certificate" may be the hash value of the immunity certificate itself. As will be described in the third example embodiment, the hash value may be stored by incorporating it into the transaction of a blockchain. Alternatively, a plurality of hash values may be collectively stored by using a hash-chaining technology. Therefore, it is possible to enhance the tolerance of an immunity certificate against tampering and the genuineness thereof by the block-chaining or hash-chaining technology.

The output unit 33 outputs the update information stored in the update information storage unit 32. For example, the output unit 33 transmits the update information to the receiving unit 22 of the information terminal 20. However, the output unit 33 may transmit the update information to other apparatuses such as the information terminal 10, or may output the update information by, for example, printing or displaying the update information.

As described above, the information system 30 records and outputs the information indicating the genuineness of the immunity certificate by using the hash value. Therefore, a person (e.g., a business proprietor) who intends to effectively use the information based on an immunity certificate can, after confirming that the immunity certificate is reliable, effectively uses the information based on the immunity certificate.

### Second Example Embodiment

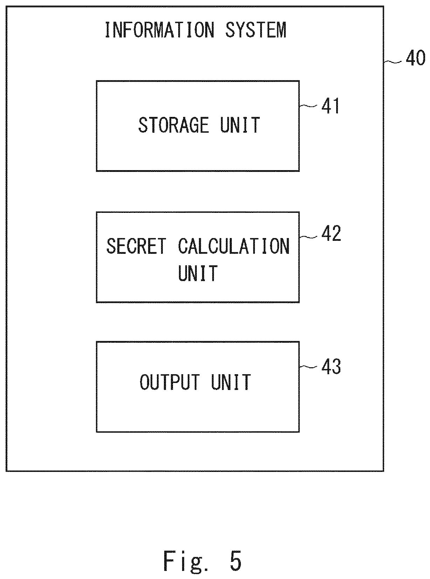

In the first example embodiment, an information system 40 may be used in place of or in combination with the information system 30. Fig. 5 is a block diagram of the information system 40. The information system 40 is a system including one or a plurality of computers (e.g., a group of servers), and includes a storage unit 41, a secret calculation unit 42, and an output unit 43.

The storage unit 41 stores an immunity certificate of one person or a plurality of immunity certificates of a plurality of persons in a format for secret calculation. Note that the "format for secret calculation" refers to a format in which secret calculation can be performed and in which each individual related to a respective immunity certificate cannot be identified from the outside. Although its specific examples will be described later, examples of the format for secret calculation include a secret sharing format and a homomorphic encryption format.

The secret calculation unit 42 performs secret calculation on an immunity certificate of one person or a plurality of immunity certificates of a plurality of person stored in the storage unit 41, and obtains a result of the secret calculation. The result of the secret calculation is, for example, the above-described "information based on an immunity certificate of an individual", and can be effectively used by a person other than the individual.

The output unit 43 outputs the result of the secret calculation performed by the secret calculation unit 42. For example, the output unit 43 transmits the update information to the receiving unit 22 of the information terminal 20. However, the output unit 33 may transmit the update information to other apparatuses such as the information terminal 10, or may output the update information by, for example, printing or displaying the update information.

As described above, the information system 40 stores the immunity certificate in the format for secret calculation, and can output the result of the secret calculation. In this way, it is possible to take the privacy of an individual into consideration and effectively use the information based on an immunity certificate at the same time.

### Third Example Embodiment

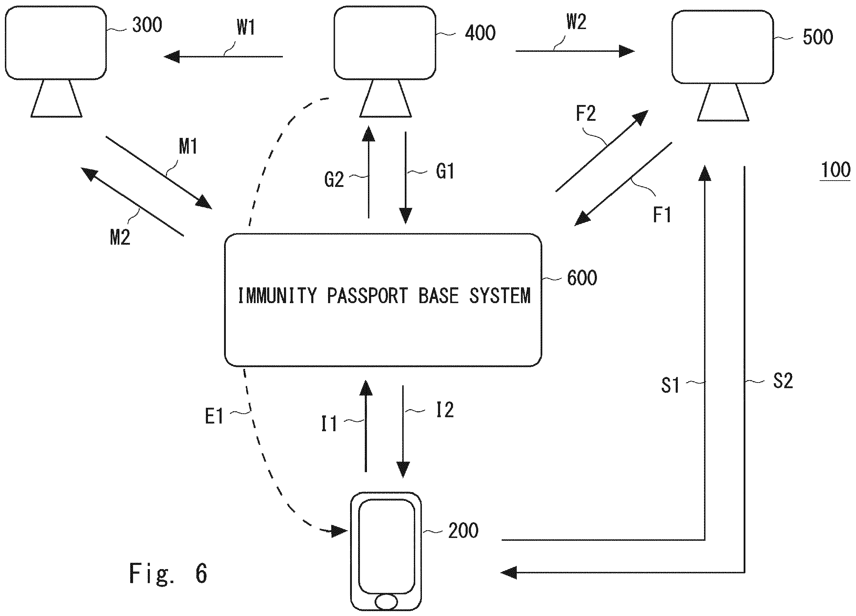

Specific examples of the immunity certificate management systems described in the first and second example embodiments will be described hereinafter. Fig. 6 is a schematic diagram of an immunity passport system according to a third example embodiment. The immunity passport system 100 includes a personal terminal 200 owned by an individual, a medical institution terminal 300 owned by a medical institution, an administrative institution terminal 400 owned by an administrative institution, a business proprietor terminal 500 owned by a business proprietor, and an immunity passport base system 600. An outline of each of the terminals and that of the base system will be described first, and then details of each component will be described.

In this example embodiment, the personal terminal 200 owned by an individual (a resident or a citizen) is a smartphone. As an example of information I1 transmitted by the personal terminal 200, the personal terminal 200 transmits immunity certificate information of an individual to the immunity passport base system 600. Examples of immunity certificate information include a location history or a movement history of an individual, a communication history with other terminals (i.e., contact information with other individuals having other terminals), biometric information, medical history information, and medication information.

The location history or movement history of an individual can be acquired, for example, by the below-described methods. That is, examples of methods include positioning by a GPS (Global Positioning System) of a smartphone, acquiring location information by a cell ID based on communication with a base station(s), a communication history with access points near a wireless LAN (Local Area Network), and a communication history with other terminals. Example of the communication through the wireless LAN include communication using Wi-Fi (Registered Trademark). Examples of the communication history with other terminals include those through short-range wireless communication means such as Bluetooth (Registered Trademark).

Further, information related to daily physical conditions or health of an individual is considered to be a type of the biometric information. The biometric information includes, for example, at least one of information items related to general vital signs such as respiration, a body temperature, a blood pressure, a pulse, the number of walking steps, and a sleeping state. The medical history information is information related to immunity to an infectious disease, such as a medical certificate issued by a doctor, vaccination, and an antibody test for an infectious disease. Further, the medication information is, for example, information related to a prescription written by a pharmacist.

Further, the personal terminal 200 may transmit, as the information I1, an acquisition request for an immunity certificate or an acquisition request for infectious-disease risk level information of a user. The "infectious-disease risk level information" is information indicating whether or not a user is suffering from a specific infectious disease or is susceptible thereto, and is information that is used by a business proprietor as a guide for determining whether or not to permit an individual to come to the office or the like, or to provide a service to an individual.

Further, the personal terminal 200 receives, from the immunity passport base system 600, information I2 including at least one of information of the immunity certificate of the user, infectious-disease risk level information (information about the result of the secret calculation for the immunity certificate), and information of the blockchain related to the immunity certificate of the user. Details of this feature will be described later.

Further, the personal terminal 200 outputs, as information S1, the infectious-disease risk level information acquired from the immunity passport base system 600 and individual identification information of the user to the business proprietor terminal 500 of the business proprietor. The individual identification information of the user is, for example, the name of the user, the My Number thereof, a photograph image thereof, and/or the like. The business proprietor terminal 500 determines, based on the information S1, whether or not to permit the user of the personal terminal 200 to come to the office or the like, or to provide a service to the user, and outputs, as information S2, the result of the determination to the personal terminal 200.

The medical institution terminal 300 owned by a medical institution (e.g., a hospital, a clinic, or a pharmacy) is a terminal that outputs immunity certificate information M1 of an individual to the immunity passport base system 600. For example, when an individual attends a hospital, a doctor who has diagnosed the individual transmits detailed diagnostic information (medical history information) of the individual to the immunity passport base system 600 by using the medical institution terminal 300. This information includes information such as whether or not he/she is infected with a specific infection, or whether or not he/she has close contact with an infected person. Further, the medical institution terminal 300 may acquire information M2 of the blockchain related to the information of the immunity certificate of the individual.

Further, the information M1 includes consent information of the individual who will be a patient in the hospital or the like. This is because since the immunity certificate is a document related to the privacy of the individual, the consent of the individual is usually required for the use of the immunity certificate. As will be described later, this consent information is also stored in the immunity passport base system 600.

Further, the medical institution terminal 300 receives a digital certificate W1 of the above-described digital signature from the administrative institution terminal 400 (which will be described later). By adding the digital signature (issuer information) using the received digital certificate W1 to the information M1 and then transmitting the information M1, the medical institution terminal 300 can prevent the information M1 from being tampered with and prove that the information M1 has been officially transmitted from the medical institution terminal 300.

The administrative institution terminal 400 owned by the administrative institution (e.g., a central government, a local government, or a local municipal government) transmits the above-described digital certificate W1 of the digital signature to the medical institution terminal 300 of the medical institution. Note that the administrative institution functions as an authentication institution that issues the digital certificate W1 only to designated medical institutions. The "designated medical institution" refers to a medical institution that is reliable enough to issue immunity certificates for specific infectious diseases.

The administrative institution terminal 400 transmits, as information G1, a request requesting to perform secret calculation based on data of a specific population (e.g., a local municipal government-level or a national-level population) to the immunity passport base system 600. The immunity passport base system 600 transmits the result of the calculation based on the request as information G2, and the administrative institution terminal 400 receives the transmitted result of the calculation. Note that, as will be described later, the result of the calculation is in such a format that the individual cannot be identified. Further, the information G2 may be information of the blockchain related to the immunity certificate of the user, for guaranteeing the validity of the result of secret calculation. Further, the administrative institution terminal 400 can transmit, as information G1, information such as the algorithm of the secret calculation performed by the immunity passport base system 600 and/or a parameter(s) used in the secret calculation.

Further, the administrative institution terminal 400 sends a notification E1 to the personal terminal(s) 200 of a specific individual(s), residents in a specific residential area(s), or all the people in the nation through the immunity passport base system 600. The notification E1 includes an alert to the infectious disease for the individual(s), the declaration of a state of emergency or the like involving the restriction on nonessential activities such as staying home, lockdowns, or the like, details of which will be described later. Further, the administrative institution terminal 400 transmits information W2, which is a public key of the digital signature, to the business proprietor terminal 500 owned by the business proprietor.

The business proprietor terminal 500 owned by the business proprietor (e.g., a company at which individuals work or a store that provides services to individuals) receives information S1 from the personal terminal 200. In response to the information S1, the business proprietor terminal 500 outputs an information request F1 to the immunity passport base system 600 in order to determine whether or not to permit the user of the personal terminal 200 to come to the office or the like, or to provide a service to the user. The information request F1 includes, in addition of the personal information of the user for whom the above-described determination is made, a request for information of the blockchain for guaranteeing the validity of the immunity certificate of the user. In response to the information request F 1, the business proprietor terminal 500 receives, as information F2, the information of the blockchain from the immunity passport base system 600. Note that the digital signature added by the medical institution terminal 300 is added to the information of the blockchain.

Further, the business proprietor terminal 500 receives information W2, which is the public key of the digital signature, from the administrative institution terminal 400 in order to determine the validity of the information of the immunity certificate acquired from the immunity passport base system 600. Based on the received information S1 and information W2, the business proprietor terminal 500 verifies whether or not the issuer of the acquired immunity certificate is the above-described designated medical institution, and whether or not the acquired immunity certificate has not been tampered with. Note that the administrative institution terminal 400 functions as an issuer management server that outputs the genuine issuer information of the digital signature.

The immunity passport base system 600 is a system that serves as a database of immunity certificates and a platform for secret calculation. The immunity passport base system 600 is operated as a neutral mechanism in which, for example, a medical institution(s), an administrative institution(s), and a telecommunication carrier(s) are stakeholders.

When the immunity passport base system 600 receives the information I1 for the immunity certificate of the individual from the personal terminal 200, the immunity passport base system 600 updates the data of the immunity certificate and the information of the blockchain stored in the storage unit by using the received data (i.e., the received information I1).

When the immunity passport base system 600 receives an acquisition request for an immunity certificate of the user, it transmits the data of the immunity certificate stored in the storage unit to the personal terminal 200 as the information I2. When the immunity passport base system 600 receives an information acquisition request for infectious-disease risk level information, it performs secret calculation and then transmits the data of the secret calculation and the information of the blockchain to the personal terminal 200 as the information I2.

The immunity passport base system 600 receives the immunity certificate information M1 of the individual from the medical institution terminal 300 of the medical institution and updates the data of the immunity certificate stored in the storage unit based on this information M1. Further, the immunity passport base system 600 may transmit the information M2 of the blockchain related to the information of the immunity certificate of the individual in response to the request from the medical institution terminal 300.

The immunity passport base system 600 receives, as information G1, a request requesting the execution of secret calculation based on data on a large number of people from the administrative institution terminal 400 of the administrative institution. Based on the request, the immunity passport base system 600 performs the secret calculation by using the data of the immunity certificate, and transmits the result of the calculation as information G2. Further, the immunity passport base system 600 may transmit, as the information G2, the information of the blockchain related to the immunity certificate of the user.

Further, when information such as the algorithm of the secret calculation and/or a parameter(s) used for the secret calculation are transmitted as the information G1, the immunity passport base system 600 updates the previously-stored information with the transmitted information. In this way, the administrative institution can update the method of the secret calculation and thereby to obtain the result of the secret calculation that is performed according to the current situation.

The immunity passport base system 600 receives an information request F1 (a request for the personal information of the user, for whom the determination is made, and the information of the blockchain for guaranteeing the validity of the immunity certificate thereof) from the business proprietor terminal 500 of the business proprietor. The immunity passport base system 600 transmits the information of the blockchain as the information F2.

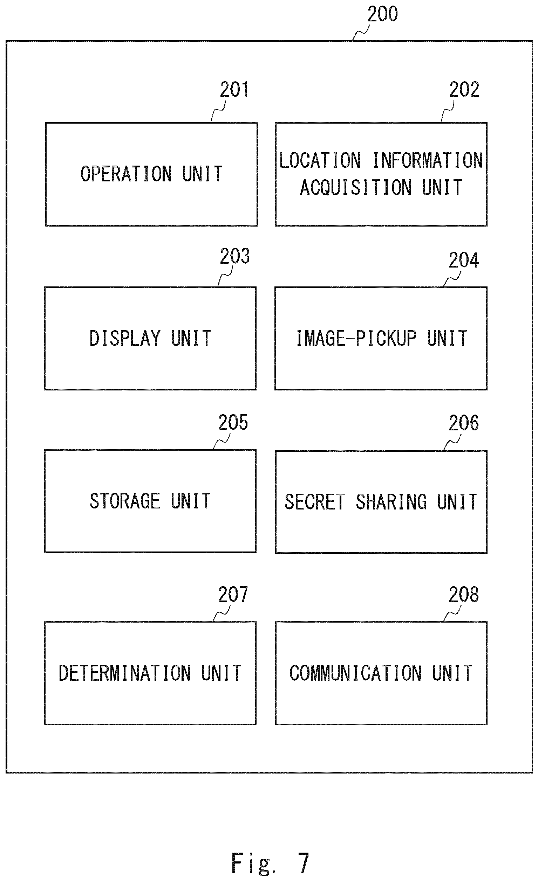

Next, details of each component of the immunity passport system 100 will be described. Fig. 7 is a block diagram showing a configuration of the personal terminal 200. The personal terminal 200 includes an operation unit 201, a location information acquisition unit 202, a display unit 203, an image-pickup unit 204, a storage unit 205, a secret sharing unit 206, a determination unit 207, and a communication unit 208.

The operation unit 201 is a component by which a user inputs information to the personal terminal 200, such as input buttons, a touch panel, and/or a speaker. By operating the operation unit 201, the user makes (i.e., instructs) the personal terminal 200 perform communication with the immunity passport base system 600. Further, the user can input immunity certificate information such as biometric information, medical history information, and medication information to the personal terminal 200 by using the operation unit 201.

The location information acquisition unit 202 is composed of at least one of a GPS function unit that performs positioning through radio communication with satellites, a cell ID identification unit based on radio communication with a base station(s), a wireless LAN communication unit that communicates with an access point(s), and a short-range wireless communication unit that communicates with other terminals. The location information acquisition unit 202 continuously or intermittently acquires location information of the personal terminal 200 by application software of the personal terminal 200, and thereby acquires a location history (a movement history) of the personal terminal 200. For example, a movement-and-contact history of the user of the personal terminal 200 can be acquired by the short-range wireless communication unit. Note that the location information acquisition unit 202 may acquire information acquired at the base station of the personal terminal 200 (i.e., acquired on the carrier side) through the communication unit 208. This information is transmitted to the immunity passport base system 600 as the information of the immunity certificate.

The display unit 203 is an example of a notification unit that notifies the user of information or the like, and includes a liquid-crystal panel, a touch panel, or the like. When the display unit 203 transmits information I1 to the immunity passport base system 600 in response to an operation performed by the user, it displays notification to request the user to consent to the transmission of the information I1. In response to the displayed notification, the user inputs his/her intention of consenting to the transmission through the operation unit 201.

Further, the display unit 203 displays information I2 including at least one of the information of the immunity certificate of the user acquired from the immunity passport base system 600, the infectious-disease risk level information, the digital signature related to the immunity certificate, and the information of the blockchain related to the immunity certificate of the user. Further, the display unit 203 can display the result of the determination (the information S2) acquired from the business proprietor terminal 500 and the notification E1 acquired from the administrative institution terminal 400.

The image-pickup unit 204 is a camera that photographs (or films) the user of the personal terminal 200. The photograph image is transmitted, as data for authenticating the user, to the business proprietor terminal 500 of the business proprietor as the information S1, and is also transmitted to the immunity passport base system 600 as the information I1.

The storage unit 205 stores, as the immunity certificate information, location information acquired by the location information acquisition unit 202, and input information such as biometric information (e.g., a body temperature), medical history information, and medication information input from the operation unit 201. The stored immunity certificate information is updated when new location information or new input information is acquired. Further, the storage unit 205 may store the infectious-disease risk level information acquired from the immunity passport base system 600 and the data of the digital signature attached thereto. Since the storage unit 205 is disposed inside the personal terminal 200, the user can display and refer to the information stored in the display unit 203 even when the personal terminal 200 is offline.

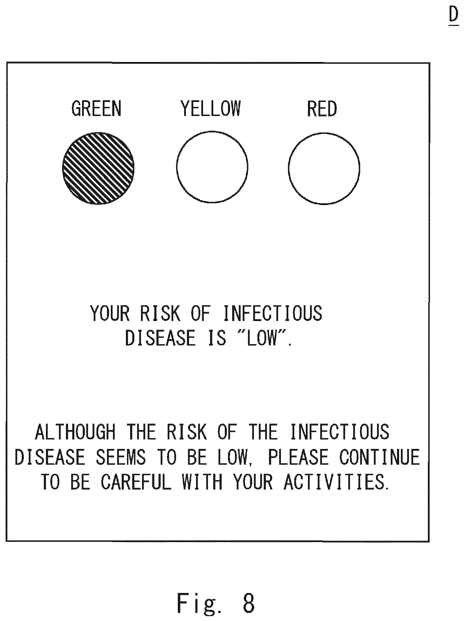

Fig. 8 shows an example of the infectious-disease risk level information stored in the storage unit 205 and the digital signature attached thereto, displayed on the display unit 203. On a screen D (e.g., a window D), as an example of the infectious-disease risk level information, the fact that the risk of the infection disease of the user is "low" is indicated by "green". Note that the infectious-disease risk level information is indicated at three levels, i.e., green, yellow, and red, in descending order of the risk. In the following description, it is assumed that these three levels are set for the infectious-disease risk level information. However, only two levels, or four or more levels, instead of the three levels, may be set for the infectious-disease risk level information.

The information stored in the storage unit 205 is transmitted as the information I1 to the immunity passport base system 600 in response to the operation performed on the operation unit 201 by the user. However, the personal terminal 200 may automatically transmit, when the immunity certificate information stored in the storage unit 205 is updated, its update information to the immunity passport base system 600 as the information I1. In this way, it is possible to synchronize the immunity certificate information of the user stored in the immunity passport base system 600 with the information stored in the personal terminal 200.

Further, when the personal terminal 200 acquires the immunity certificate information of the user from the immunity passport base system 600, it may synchronize the information stored in the storage unit 205 with the acquired immunity certificate information.

The secret sharing unit 206 secretly shares (i.e., secretly divides or distributes) the information stored in the storage unit 205 while incorporating the consent history of the user thereinto. This process is performed in order to (i) prevent the individual from being identified from the information I1, and (ii) facilitate the secret calculation performed in the immunity passport base system 600. The secret sharing unit 206 outputs the information I1, which has been encrypted as it has been secretly shared (i.e., secretly divided or distributed), to the communication unit 208.

When the determination unit 207 acquires the immunity certificate information of the user or the infectious-disease risk level information thereof from the immunity passport base system 600, it refers to a hash value contained in the transaction of the blockchain added to the aforementioned information, and thereby determines whether the acquired data has been tampered with. When the hash value is normal, it is determined that the acquired data has not been tampered with and hence is genuine. On the other hand, when the hash value is abnormal, it is determined that the acquired data has been tampered with and hence is incorrect.

The communication unit 208, which is a radio communication unit, transmits, to the immunity passport base system 600, the information of the immunity certificate, which has been secretly shared, and also transmits an acquisition request for an immunity certificate, infectious-disease risk level information, or the like. Further, the communication unit 208 receives the information I2 from the immunity passport base system 600. The wireless communication is carried out through a base station (not shown) through a wireless LAN or the like. When the intention of consenting to the transmission is input through the operation unit 201, the communication unit 208 transmits the information I1, which has been secretly shared, to the immunity passport base system 600. Further, the communication unit 208 transmits the information S1 to the business proprietor terminal 500 of the business proprietor, and also receives the result of the determination (the information S2) from the business proprietor terminal 500. Further, the communication unit 208 can acquire the notification E1 from the administrative institution terminal 400.

Further, the communication unit 208 may perform a pseudonymization process on the information I1 when it transmits the information I1 to the immunity passport base system 600. By associating the information I1 with a pseudonymized ID (a pseudonym ID), it is possible to prevent the individual from being identified from the information M1. Although the pseudonymized ID is associated with an individual in a one-to-one manner, the individual cannot be identified from the pseudonymized ID. For example, although the pseudonymized ID is determined based on a number for identifying the individual (such as a My Number or a Social Security Number), the My Number cannot be inversely-calculated from the pseudonymized ID.

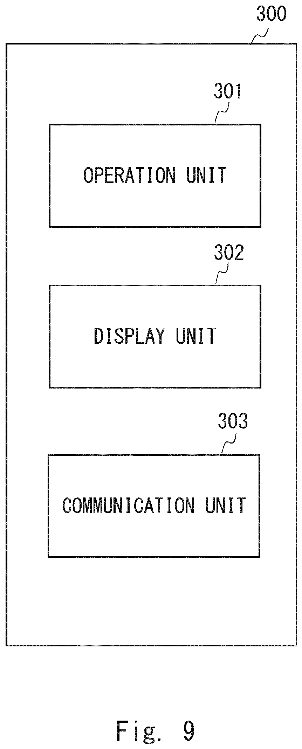

Fig. 9 is a block diagram showing a configuration of the medical institution terminal 300. The medical institution terminal 300 includes an operation unit 301, a display unit 302, and a communication unit 303. Details of each unit will be described hereinafter.

The operation unit 301 is a component by which the user inputs information to the medical institution terminal 300, such as a keyboard, input buttons, and/or a touch panel. By operating the operation unit 301, the user makes (i.e., instructs) the medical institution terminal 300 communicate with the immunity passport base system 600. Further, the user can, by using the operation unit 301, input the above-described information M1 to the medical institution terminal 300 while adding the digital certificate W1 thereto.

The display unit 302 includes a liquid-crystal panel, a touch panel, or the like, and enables the user to see the input operation performed on the operation unit 301.

The communication unit 303 transmits the information M1 for the immunity certificate of the individual and the consent thereto to the immunity passport base system 600, and also receives the above-described digital certificate W1 of the digital signature from the administrative institution terminal 400. Further, the communication unit 303 can receive the information M2 of the blockchain related to the information of the immunity certificate of the individual.

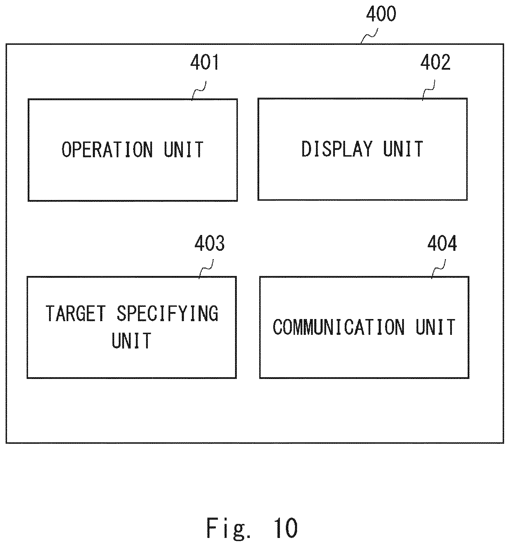

Fig. 10 is a block diagram showing a configuration of the administrative institution terminal 400. The administrative institution terminal 400 includes an operation unit 401, a display unit 402, a target specifying unit 403 and a communication unit 404. Details of each unit will be described hereinafter.

The operation unit 401 is a component by which the user inputs information to the administrative institution terminal 400, such as a keyboard, input buttons, and/or a touch panel. By operating the operation unit 401, the user makes (i.e., instructs) the administrative institution terminal 400 communicate with the personal terminal 200, the medical institution terminal 300, the business proprietor terminal 500, or the immunity passport base system 600. The display unit 402 includes a liquid-crystal panel, a touch panel, or the like, and enables the user to see the input operation performed on the operation unit 401.

The target specifying unit 403 specifies the target to which the notification E1 should be issued based on the result of the calculation performed based on the request acquired from the immunity passport base system 600 (i.e., based on the information G2). For example, when the result of the calculation indicates a group of people who have visited a specific facility where a patient having the infectious disease has occurred (e.g., has been found), the target specifying unit 403 issues an alert to the terminals of the individuals belonging to the group and instructs them to refrain from doing nonessential activities or to take a test.

Further, the target specifying unit 403 may be configured so that when the result of the calculation indicates the prevalence of the infectious disease, such as the number of patients of the infectious disease or the number of tests for the infectious disease in a certain residential area(s) or in the whole nation, it sends, based on the result of the calculation, a notification about the declaration of a state of emergency or the like to the terminals of residents or the people in the nation. Note that one of the features of the administrative institution terminal 400 is that it cannot identify any specific individual among the people or the like specified by the target specifying unit 403.

Further, the target specifying unit 403 may determine the validity of the acquired result of the calculation based on the information of the blockchain related to the immunity certificate of the user. As will be described later, the hash value contained in the transaction of the blockchain indicates a normal value unless the information of the immunity certificate stored in the immunity passport base system 600 has been tampered with. Therefore, when all or almost all of the hash values related to the users for which the calculation is performed indicate normal values, the target specifying unit 403 determines that the result of the calculation is valid. However, if this is not the case, it means that the original data of the calculation has been tampered with, so that the target specifying unit 403 determines that the result of the calculation is inappropriate.

The communication unit 404 transmits, as the information G1, information such as a request for secret calculation, the algorithm and a parameter(s) used for the secret calculation to the immunity passport base system 600. The communication unit 404 receives, as the information G2, the result of the calculation based on the request from the immunity passport base system 600. Further, the communication unit 404 sends the notification E1 to the target terminals specified by the target specifying unit 403 through the immunity passport base system 600. Further, the communication unit 404 transmits the information W2, which is the public key of the digital signature, to the business proprietor terminal 500 owned by the business proprietor. Note that the administrative institution terminal 400 functions as an issuer information management server that indicates that it is the genuine issuer of the digital signature.

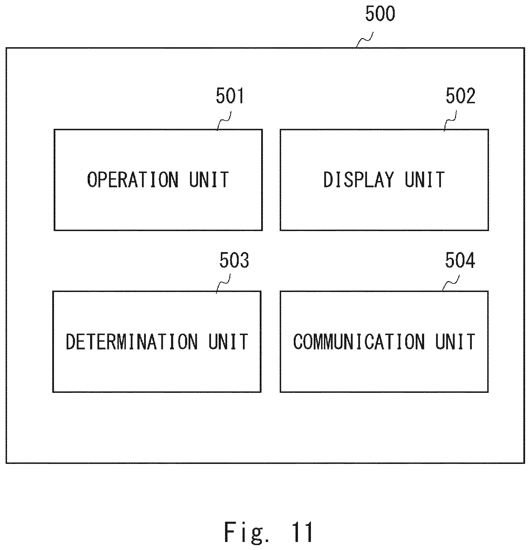

Fig. 11 is a block diagram showing a configuration of the business proprietor terminal 500. The business proprietor terminal 500 includes an operation unit 501, a display unit 502, a determination unit 503 and a communication unit 504. Details of each unit will be described hereinafter.

The operation unit 501 is a component by which the user inputs information to the business proprietor terminal 500, such as a keyboard, input buttons, and/or a touch panel. By operating the operation unit 501, the user makes (i.e., instructs) the business proprietor terminal 500 communicate with the personal terminal 200, the administrative institution terminal 400, or the immunity passport base system 600. The display unit 402 includes a liquid-crystal panel, a touch panel, or the like, and enables the user to see the input operation performed on the operation unit 401.

When the determination unit 503 receives the information S1 from the personal terminal 200, it determines whether or not to permit the user of the personal terminal 200 to come to the office or the like, or to provide a service to the user according to the information S1. For the determination, the determination unit 503 acquires, from the immunity passport base system 600, the information of the immunity certificate of the user, for which the determination is made, and the information of the blockchain for guaranteeing the validity of the immunity certificate thereof. Note that the information of the blockchain includes the digital signature added by the medical institution terminal 300. Further, the determination unit 503 receives the information W2, which is the public key of the digital signature, from the administrative institution terminal 400.

The determination unit 503 determines whether the digital signature acquired from the immunity passport base system 600 corresponds to the public key of the digital signature acquired from the administrative institution terminal 400. When the digital signature corresponds to the public key, the determination unit 503 determines that the immunity certificate of the user has been issued by the designated medical institution designated by the administrative institution. When the digital signature does not correspond to the public key, the determination unit 503 determines that the immunity certificate of the user is not the one that has been issued by the designated medical institution designated by the administrative institution.

Further, the determination unit 503 refers to the information (the hash value) contained in the transaction of the blockchain for guaranteeing the validity of the immunity certificate, and thereby verifies whether or not the acquired immunity certificate has been tampered with. When the hash value is a normal value, the determination unit 503 determines that the immunity certificate of the target individual has not been tampered with. On the other hand, when the hash value is an abnormal value, the determination unit 503 determines that the immunity certificate of the target individual has been tampered with.

When it is determined, in the above-described determination, that the immunity certificate of the user has been issued by the designated medical institution and has not been tampered with, the determination unit 503 determines that the infectious-disease risk level information of the user is valid. If this is not the case, the determination unit 503 determines that the infectious-disease risk level information of the user is invalid. When it is determined that the infectious-disease risk level information of the user is valid, the determination unit 503 approves (i.e., permits) the user of the personal terminal 200 to come to the office or the like, or approves to provide a service to the user. On the other hand, when it is determined that the infectious-disease risk level information of the user is invalid, the determination unit 503 does not approve the user of the personal terminal 200 to come to the office or the like, or does not approve to provide a service to the user.

Further, when the infectious-disease risk level information is transmitted from the personal terminal 200 and the immunity certificate of the user is determined to be valid, the determination unit 503 may further verify the contents of the infectious-disease risk level information. When it is determined that the risk of the infection or development of the infectious disease of the user is low (because, for example, the user has already acquired the immunity to the infectious disease) and hence the infectious disease risk level is "green," the determination unit 503 approves the user of the personal terminal 200 to come to the office or the like, or approves to provide a service to the user. In addition to this case, when the risk is lower than or equal to a predetermined threshold (e.g., the risk is lower than or equal to "yellow"), the determination unit 503 may give similar approval.

The communication unit 504 receives, as the information S1, information such as infectious-disease risk level information and individual identification information of the user from the personal terminal 200. Further, the communication unit 504 outputs the result of determination made by the determination unit 503 to the personal terminal 200 as the information S2.

Further, the communication unit 504 receives the information W2, which is the public key of the digital signature, from the administrative institution terminal 400. Further, the communication unit 504 transmits, to the immunity passport base system 600, the information request F1 requesting information that the determination unit 503 will use for the determination. The communication unit 504 receives, as the information F2, the information of the immunity certificate and the information of the blockchain from the immunity passport base system 600.

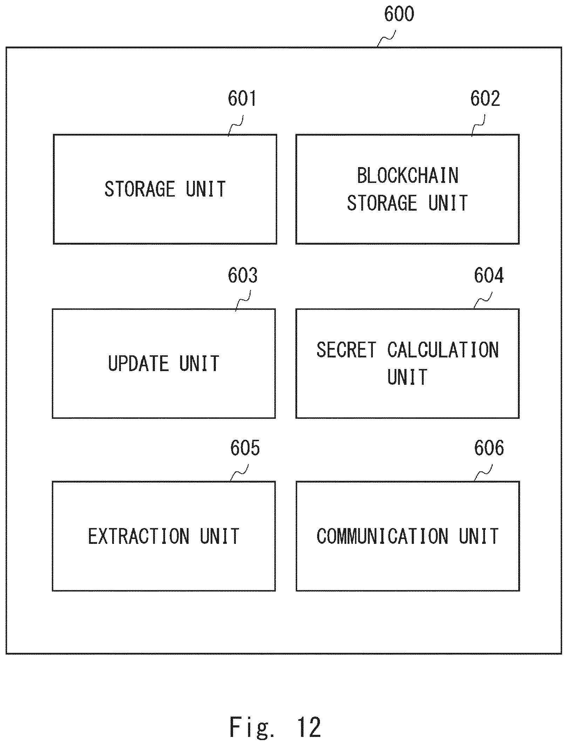

Fig. 12 is a block diagram showing a configuration of the immunity passport base system 600. The immunity passport base system 600 is composed of a plurality of servers, and includes a storage unit 601, a blockchain storage unit 602, an update unit 603, a secret calculation unit 604, an extraction unit 605, and a communication unit 606. Details of each unit will be described hereinafter.

The storage unit 601 is composed of a plurality of storage units distributed over a plurality of servers, and stores (registers) a number of immunity certificates of a number of users while associating them with pieces of contact information of respective personal terminals 200 of the users. The immunity certificate of each individual is stored in a secretly-shared format over a plurality of storage units (i.e., in a fragment format so-called "share"). Therefore, even when the storage unit 601 is accessed from the outside of the immunity passport base system 600, only randomly shared data can be acquired, so that the immunity certificate of the individual cannot be reproduced. That is, it is impossible to identify the individual.

As an example, the storage unit 601 stores the below-shown information.
(a) Movement-and-contact history of each individual
(b) Body-temperature history of each individual
(c) Antibody test result or the like of infectious disease of each individual Among these information items, the information items (a) and (b) are transmitted from the personal terminal 200 of the individual as the information I1, and the information item (c) is transmitted from the medical institution terminal 300 of the medical institution as the information M1.

The blockchain storage unit 602 stores, by using the block-chaining technology, an update history of the immunity certificate in association with the immunity certificate stored in the storage unit 601. The update history of the immunity certificate stored in the storage unit 601 is stored in the blockchain storage unit 602 as a hash value (i.e., in the form of a hash value). When secret calculation (which will be described later) is performed by using the immunity certificate, a link between the hash value and the secret calculation is generated.

As an example, the blockchain storage unit 602 stores the below-shown information.
- Hash value of individual consent history and digital signature of each individual
- Hash value of movement-and-contact history and digital signature of each individual
- Hash value of body-temperature history and digital signature of each individual
- Hash value of medical history/diagnostic detail and digital signature of each individual
Note that the individual consent history corresponds to the information related to the consent transmitted by the personal terminal 200 or the medical institution terminal 300.

When the update unit 603 receives the immunity certificate information I1 from the personal terminal 200 or the immunity certificate information M1 from the medical institution terminal 300 of the medical institution, it updates the immunity certificate stored in the storage unit 601 by using the received information. When doing so, the update unit 603 stores the update history in the blockchain storage unit 602 as the above-described hash value. Further, the digital signature attached to the information M1 is also stored in the blockchain storage unit 602.

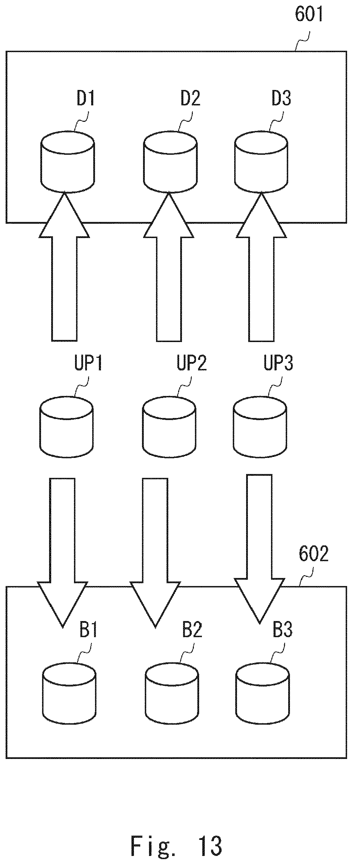

Fig. 13 is an image of processing performed by the update unit 603. When the update unit 603 acquires update information pieces UP1 to UP3 related to the immunity certificate from the personal terminal 200 or the medical institution terminal 300, it updates data D1 to D3 of the immunity certificate, which are stored in the storage unit 601 in a secretly sharing manner, by using these data (i.e., these information pieces). Further, the update unit 603 updates update history data B1 to B3 corresponding to the data D1 to D3 by using the hash values generated as results of the updating and the digital signatures of the designated medical institution contained in the update information pieces UP1 to UP3. Note that the data D1 to D3 and the update history data B1 to B3 are stored in respective servers, i.e., in three servers, in a shared manner.

The description is continued by referring to Fig. 12 again. The secret calculation unit 604 performs secret calculation by using the immunity certificate of the user stored in the storage unit 601 as the original data. The secret calculation unit 604 is composed of a plurality of information processing units distributed over a plurality of servers, and performs secret calculation for a number of immunity certificates of a number of users. Regarding the process of this secret calculation, the secret calculation is performed by having the plurality of information processing units cooperate with each other without reproducing the original contents of the immunity certificate of the user.

For example, assume that the immunity passport base system 600 is composed of three servers as described above, and a number of immunity certificates of a number of users are secretly shared (i.e., secretly divided) into data D1, D2 and D3. In this state, the secret calculation unit 604 acquires the final result of the secret calculation by performing a calculation process for each of the data D1, D2 and D3 and combining the results of these three calculation processes.

There are two types of secret calculation performed by the secret calculation unit 604, i.e., (i) secret calculation based on an acquisition request for infectious-disease risk level information transmitted from the personal terminal 200 of the individual, and (ii) secret calculation based on one transmitted from the medical institution terminal 300 of the administrative institution. However, in both the secret calculation (i) and the secret calculation (ii), the algorithm and a parameter(s) used in the secret calculation are stored in advance in the secret calculation unit 604 and updated by the information G1 according to the intention of the administrative institution. Each type of calculation will be described hereinafter.
(i) When an acquisition request for infectious-disease risk level information is transmitted from the personal terminal 200 of the individual, the secret calculation unit 604 specifies the individual, for whom the result of the calculation is obtained, based on the personal information contained in the acquisition request. After that, the secret calculation unit 604 performs secret calculation by using the immunity certificate of the user stored in the storage unit 601, and calculates infectious-disease risk level information of the specified individual by using the result of the calculation and the personal information.

The original data of the calculated infectious-disease risk level information is information such as physical condition information of the individual, medical history information thereof, and movement history information thereof. Therefore, when the physical condition of the individual or his/her movement history changes, the risk level indicated by the infectious-disease risk level information changes. For example, when a given individual has had a temperature higher than 37.3 degrees for two consecutive days, it is determined that the probability of the individual having the infectious disease has increased. As an example, the risk level, which has been originally indicated by green or yellow, changes to red. In this case, the business proprietor may determine to refuse this individual to enter the facility or the like.

Further, the risk level of the infectious disease also changes when a mutation occurs in the virus of the infectious disease. For example, when an individual has contracted a specific virus (hereinafter also referred to as a virus A) and hence is immune to the virus A, his/her infection risk level is "green". However, when a virus B, which appears by a mutation of the virus A, is going around, the risk level of the individual of the infection for the virus B becomes "yellow" or "red". This is because, in the algorithm for the secret calculation, the risk of infection is rated higher because the individual is not immune to the virus B.

Further, in the secret calculation, it is also possible to determine whether a specific individual has been in close proximity or in contact with an infected person or a close contact person of an infected person (a contact history between individuals) based on the data of the movement history of people using, for example, a GPS or short-range wireless communication between terminals. Information as to whether a given person is an infected person or a close contact person of an infected person is included in the data of the immunity certificate as infected-person information. Alternatively, it may be determined whether a specific individual has visited an infectious-disease occurrence facility (e.g., a cluster occurrence source). The risk level indicated by the infectious-disease risk level information changes in response to the above-described calculation.

(ii) When an execution request for secret calculation is transmitted from the administrative institution terminal 400 of the administrative institution, the secret calculation unit 604 performs secret calculation for a specific population specified in the execution request by using immunity certificates of users stored in the storage unit 601. This secret calculation is an analytical process that is carried out to, for example, find out the number of tests and the number of infected people in a specific area(s), or to enable the administrative institution to find out how much the herd immunity has been achieved. Based on the result of the secret calculation, the administrative institution issues an alert against the infectious disease to individuals. Further, the administrative institution may, after examining a threshold that serves as an indicator for the declaration of a state of emergency or the like, issues the declaration of a state of emergency or the like involving the restriction on nonessential activities such as staying home, lockdowns, or the like.

For example, when the number of people whose risk level is indicated by red is larger than or equal to a predetermined percentage of the population, or when the increase in the number of people over a predetermined period becomes larger than or equal to a predetermined rate, the administrative institution issues the declaration of a state of emergency in the target area(s). Note that, as an example, the percentage of positive cases (number of infected people) to the number of tests, instead of the number of people whose risk level is indicated by red, may be used as the indicator. In this case, the population of the secret calculation is the people who undergo tests, and the result extracted in the secret calculation is the percentage of positive cases.

Further, the secret calculation performed based on the execution request may be one for specifying close contact persons of infected persons (persons suspected of being infected) from among the population. The close contact person may be, for example, a person who has been with an infected person within a predetermined distance and for a predetermined time or larger, or a person who has visited am infectious-disease occurrence facility. Similarly to the above-described secret calculation, this secret calculation is performed based on the data of the movement history.

As described above, the results of these calculations are transmitted to the administrative institution terminal 400 as the information G2. Further, information about the result of the calculation is stored in the memory of the immunity passport base system 600. Based on the information G2, the target specifying unit 403 of the administrative institution terminal 400 issues the notification E1, through the immunity passport base system 600, to a person(s) determined to be a close contact person(s), or to residents or the like in a specific area(s), extracted from the result of the calculation. Note that, on the administrative institution terminal 400 side, it is impossible to identify the specific individual to whom the notification E1 is transmitted. The immunity passport base system 600 transfers the notification E1 to the target personal terminal 200 by using the information stored in the memory and the contact information of the personal terminal 200 of each of users associated with respective immunity certificates. The notification E1 may be, for example, an alert to a close contact person, urging him/her to refrain from doing nonessential activities or to take a test, or may be the declaration of a state of emergency to residents in a specific area.

Further, the result of the secret calculation in the item (ii) may be transmitted from the immunity passport base system 600 to other information terminals. The result itself is not information by which the privacy of the individual is violated, but is useful information for the infectious disease and hence can be laid open to the public.

The description is continued by referring to Fig. 12 again. When an information request F1 is transmitted from the business proprietor terminal 500, the extraction unit 605 extracts the information of the blockchain of the target user, stored in the blockchain storage unit 602 based on the personal information of the user contained in the information request F1. This information includes the digital signature and the hash value as described above.

The communication unit 606 transmits and receives the information items I1, I2, M1, M2, G1, G2, F1 and F2, and the notification E1 to and from each terminal.

For example, when the secret calculation unit 604 has calculated infectious-disease risk level information of a specific individual in the item (i), the communication unit 606 adds, from among the data stored in the blockchain storage unit 602, the hash value and digital signature corresponding to that individual to the calculated information. The resultant information of this process is transmitted to the personal terminal 200 of the individual as the information I2.

Further, when the secret calculation unit 604 performs secret calculation based on a request from the administrative institution terminal 400 in the item (ii), the communication unit 606 adds a hash value and a digital signature corresponding to the result of this secret calculation to the calculated information. The resultant information of this process is transmitted to the administrative institution terminal 400 as the information G2.

Further, when the notification E1 is received from the administrative institution terminal 400, as described above, the communication unit 606 transmits the notification E1 to the target personal terminal 200 by using the information about the result of the calculation and the contact information of the personal terminal 200 of each of users associated with respective immunity certificates.

Further, when the information request F1 is received from the business proprietor terminal 500, the communication unit 606 transmits the information F2 of the blockchain of the user, extracted by the extraction unit 605 to the business proprietor terminal 500.

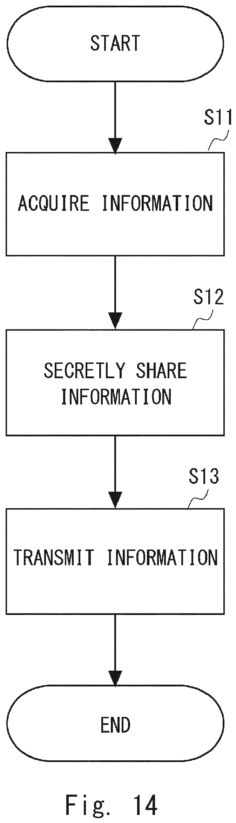

Typical processes performed by the personal terminal 200, the business proprietor terminal 500, and the immunity passport base system 600 will be described hereinafter again by using a flowchart. Firstly, processes performed by the personal terminal 200 will be described with reference to Fig. 14.

Firstly, the personal terminal 200 acquires immunity certificate information (S11). As described above, examples of the method for acquiring immunity certificate information include entering information through buttons and the like, photographing, and acquiring online.

The secret sharing unit 206 of the personal terminal 200 secretly shares (i.e., secretly divides or distributes) the acquired immunity certificate information before transmitting it to the immunity passport base system 600 (S12).

The communication unit 208 transmits the secretly-shared immunity certificate information to the immunity passport base system 600 (S13).

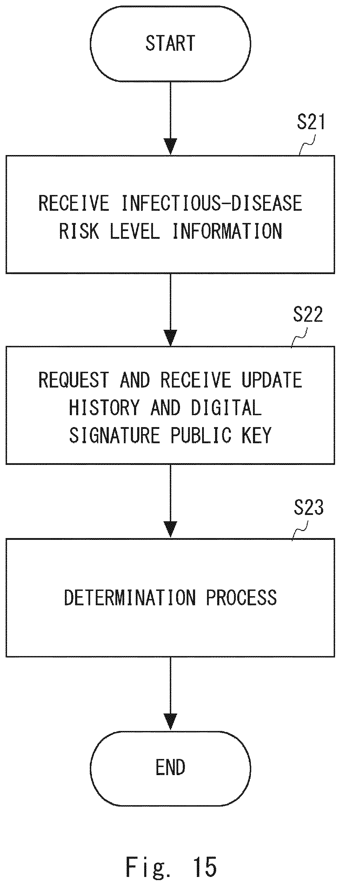

Next, processes performed by the business proprietor terminal 500 will be described with reference to Fig. 15. Firstly, the communication unit 504 of the business proprietor terminal 500 receives infectious-disease risk level information from the personal terminal 200 (S21).

After that, the business proprietor terminal 500 requests the information of the update history (the hash value and the digital signature) of the immunity certificate corresponding to the infectious-disease risk level information from the immunity passport base system 600. Further, the business proprietor terminal 500 requests public key information corresponding to the digital signature information from the administrative institution terminal 400. The communication unit 504 of the business proprietor terminal 500 receives these information items which have been output according to the requests (S22).

Then, the determination unit 503 of the business proprietor terminal 500 determines whether or not the immunity certificate has been tampered with by using the hash value of the immunity certificate. Further, the determination unit 503 determines whether or not the digital signature information is one issued by the designated medical institution by using the public key information (S23). In this way, the determination unit 503 determines whether or not the infectious-disease risk level information of the user is valid.

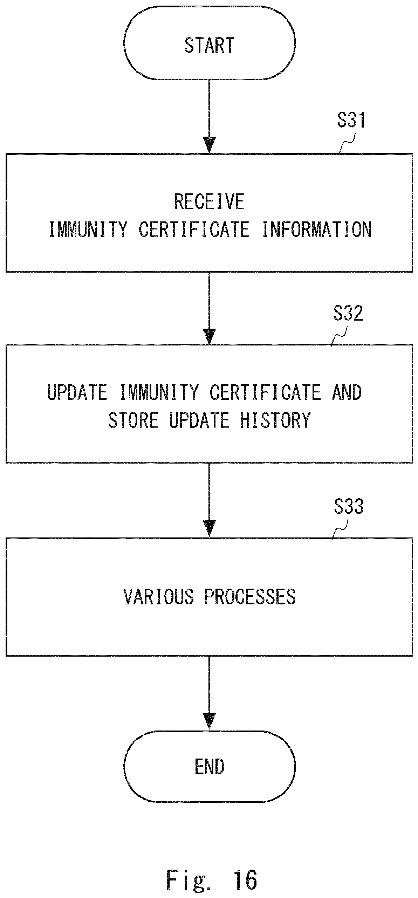

Next, processes performed by the immunity passport base system 600 will be described with reference to Fig. 16. Firstly, the communication unit 606 of the immunity passport base system 600 receives immunity certificate information from the personal terminal 200 or the medical institution terminal 300 (S31).

Note that when the immunity passport base system 600 receives immunity certificate information, it sends, to the personal terminal 200 or the medical institution terminal 300, a consent request for the transmission of the immunity certificate information. In this way, the individual or the like is notified of a caution about the transmission of the immunity certificate information, which is personal information, through a display unit, a sound unit, a vibration function, or the like of the personal terminal 200 or the medical institution terminal 300. Then, after receiving the consent information from the personal terminal 200 or the medical institution terminal 300, the immunity passport base system 600 starts receiving the immunity certificate information.

Note that the immunity passport base system 600 can output information for notifying the individual or the like of a caution about the transmission of the immunity certificate information, which is personal information, as well as the consent request.

The update unit 603 updates the immunity certificate stored in the storage unit 601 in a secretly-sharing format by using the received the immunity certificate information. That is, the immunity certificate information is used as the update data. Further, the update history is stored in the blockchain storage unit 602 (S32).

As described above, the immunity passport base system 600 performs various processes by using the immunity certificate or the update history stored in the immunity passport base system 600 (S33). For example, when the immunity passport base system 600 receives a request for secret calculation from the personal terminal 200 or the administrative institution terminal 400, it performs the secret calculation in the secret calculation unit 604 and outputs the result of the secret calculation to the terminal or the like which has issued the request. Further, when the information request F1 is transmitted from the business proprietor terminal 500, the extraction unit 605 extracts and outputs the update history of the target user stored in the blockchain storage unit 602.

To effectively use immunity certificates, a company or a facility needs to check whether an immunity certificate possessed by an individual is genuine or not. However, ensuring the genuineness of an immunity certificate through an anti-forgery printing technology is troublesome and involves risks such as a risk of theft. Further, acquiring an immunity certificate online involves a risk of tampering.

Further, when an administrative institution uses data of current health information of residents, the data they use needs to be genuine for guaranteeing the correctness of the prediction. Further, it is desired to take measures as much as possible to prevent information based on which the individual can be identified from being known by (i.e., being leaked to) a governmental institution or other third parties.

In the third example embodiment, the immunity passport base system 600 stores immunity certificates in the storage unit 601 in such a manner that secret calculation can be performed thereon, so that the immunity certificates cannot be viewed from outside (e.g., from an administrative institution or a business proprietor) except for from the personal terminal 200 of the individual. Therefore, only the individual can find (e.g., view) his/her immunity certificate, thus making it possible to protect his/her privacy.

Further, the result of the secret calculation of the immunity certificate performed by the secret calculation unit 604 is supplied to the administrative institution terminal 400 while the contents of the immunity certificate is kept secret. Therefore, even though immunity certificates of individuals are not provided, the governmental institution can acquire the necessary calculation result. To work out (i.e., to determine) how much social activities should be allowed, the administrative institution needs to predict the current and future infection situation or economic activities by using the data of the current health information of residents. By the result of this calculation, the administrative institution can accurately assess (or predicts), for example, the situation of herd immunity and the sign of the prevalence of the infectious disease while obtaining public understanding and taking their privacy into consideration.

Further, the immunity passport base system 600 can provide infectious-disease risk level information to each terminal based on the stored immunity certificate. In this way, it is possible to lift activity restraint or activity restriction for individuals at low risk of the infectious disease.

Further, since the blockchain storage unit 602 stores the update history of the immunity certificate as the hash value, it is easy to determine whether or not the immunity certificate has been tampered with. Further, the blockchain storage unit 602 also stores the data of digital signatures, it is also easy to determine whether or not the immunity certificate has been issued by a reliable designated medical institution.

As an example, when the business proprietor is a restaurant owner, the business proprietor can determine, by the above-described determination process, whether or not to permit both the employees (clerks) and customers to enter the restaurant by using the infectious-disease risk level information. In this way, it is possible to control the spread of the infectious disease and enable the business proprietor to continue his/her business (economic activities) at the same time.

Further, since the data on immunity certificates are stored through the secret calculation and the blockchain storage unit 602 records only their hash values, a large number of data can be stored. Therefore, the scalability of the immunity passport base system 600 is improved. Further, by storing the hash value by containing it in the transaction of the blockchain, the tolerance against data tampering can be enhanced.

The personal terminal 200 can provide information of the immunity certificate to the user as a PDS (Personal Data Service), and can provide infectious-disease risk level information to the user. In this way, the user can collectively manage management information related to the infectious disease with one personal terminal 200.

The immunity passport base system 600 makes the personal terminal 200 or the medical institution terminal 300 to notify the individual or the like of a caution about the transmission of the immunity certificate information when it receives the immunity certificate information therefrom. In this way, it is possible to effectively use the immunity certificate under the understanding of persons concerned.

### Fourth Example Embodiment

In the above-described third example embodiment, the immunity passport base system 600 sends a consent request to the personal terminal 200 or the medical institution terminal 300 when the immunity passport base system 600 receives the immunity certificate information. The immunity passport base system 600 may authenticate the user instead of or in combination with the aforementioned consent request.

Specifically, the immunity passport base system 600 holds the personal identification number or the number assigned to the designated medical institution related to the individual of the immunity certificate, and when the immunity passport base system 600 receives such information from the personal terminal 200 or the medical institution terminal 300, it may compare the received information with the information held therein. When the result of the comparison is appropriate, the immunity passport base system 600 acquires the immunity certificate information from the personal terminal 200 or the medical institution terminal 300. On the other hand, when the result of the comparison is inappropriate, the immunity passport base system 600 does not acquire the immunity certificate information from the personal terminal 200 or the medical institution terminal 300. In this way, it is possible to prevent the immunity certificate from being tampered with and from being mixed with immunity certificates of others.

### Fifth Example Embodiment

The storage unit 601 of the immunity passport base system 600 stores the immunity certificate of each individual in a secretly-shared format. However, the storage unit 601 may store the immunity certificate of each individual in other formats as long as the format is one for secret calculation.

For example, the storage unit 601 stores the immunity certificate of each individual in a homomorphic encryption format. When the administrative institution terminal 400 requests secret calculation, it transmits a ciphertext indicating the request to the immunity passport base system 600. Based on the ciphertext received by the communication unit 606, the secret calculation unit 604 performs the secret calculation while keeping the immunity certificate encrypted. The obtained result of the secret calculation is transmitted from the communication unit 606 to the administrative institution terminal 400 in the encrypted state. The administrative institution terminal 400 decrypts the encrypted result and acquires the result of the calculation.

Further, the storage unit 601 may store the immunity certificate of each individual in an encrypted format, and the secret calculation unit 604 may secretly calculate the data to be calculated (the immunity certificate of each individual) in an area that is inaccessible from outside terminals (i.e., in the so-called "safe area"). The safe area is, for example, an Enclave area of the memory of the computer that constitutes the immunity passport base system 600. Within this safe area, the immunity passport base system 600 decrypts the immunity certificate of each individual and performs the calculation, and then transmits the result of the calculation from the communication unit 606 to the administrative institution terminal 400.

### Sixth Example Embodiment

In the third example embodiment, the personal terminal 200 is a smartphone. However, the personal terminal 200 may be any of other types of terminals as long as it can communicate with the immunity passport base system 600 through the Internet. For example, the information terminal may be any of other types of wearable information terminals.

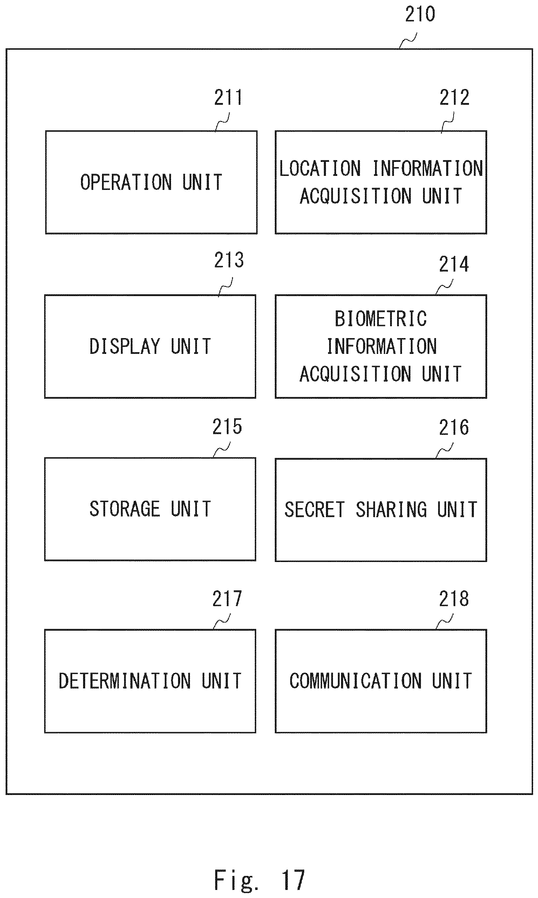

Fig. 17 is a block diagram of an information terminal in the case where the information terminal is a smartwatch as an example of the wearable information terminal. The personal terminal 210 includes an operation unit 211, a location information acquisition unit 212, a display unit 213, a biometric information acquisition unit 214, a storage unit 215, a secret sharing unit 216, a determination unit 217, and a communication unit 218.

The biometric information acquisition unit 214 includes a detection unit such as an electric-signal sensor or an optical sensor, and acquires a vital sign(s) of the user who is wearing the personal terminal 210. The acquired vital sign is secretly shared by the secret sharing unit 216, and then transmitted to the immunity passport base system 600 through the communication unit 218. Note that, in the transmission, a user consent or user authentication process is performed as described above. Note that the processes performed by the components other than the biometric information acquisition unit 214 are similar to those performed in the above-described personal terminal 200, and therefore descriptions thereof are omitted.

The determination unit 217 may have a digital signature verification function in addition to making a determination as to the abnormality of the hash value. The method for verifying the digital signature is the same as the above-described method, and therefore details thereof are omitted. The public key necessary for the digital signature can be acquired from the administrative institution terminal 400 of the administrative institution.

As described above, by providing the personal terminal 210 with the biometric information acquisition unit 214, it is possible to update the information of the immunity certificate without having the user input his/her biometric information by himself/herself.

### Seventh Example Embodiment

Further, the personal terminal 200 may be an ordinary PC (Personal Computer) including a keyboard as an input unit. In this case, the communication unit 208 may be a communication unit that performs communication through a wired LAN. The user can make the personal terminal 200 acquire a location history or movement history of the individual manually or through other terminals.

Further, a personal information management system such as a personal data store or an information bank may be used in place of the personal terminal 200.

### Eighth Example Embodiment

Further, the medical institution terminal 300 may include components similar to those of the secret sharing unit 206 of the personal terminal 200. The medical institution terminal 300 may secretly share the information M1 by the secret sharing unit and then transmit it by the communication unit 303, so that it can prevent privacy data or the like in the information M1 from being leaked.

### Ninth Example Embodiment

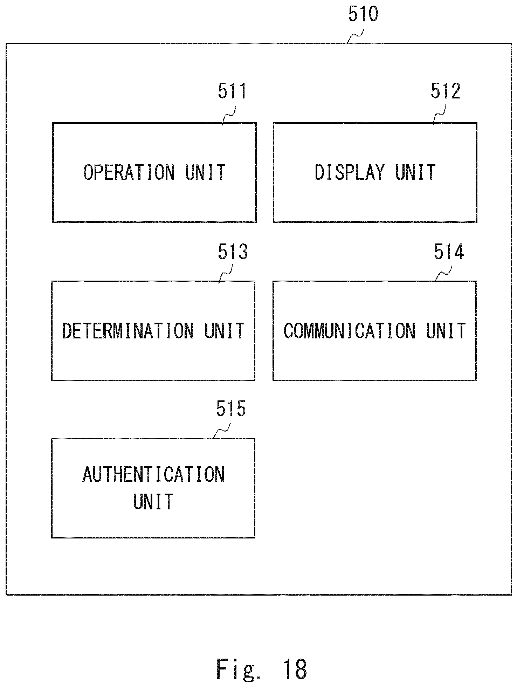

The business proprietor may determine, after authenticating the user who owns the personal terminal 200, whether or not to permit the user to come to the office or the like, or provide a service. Fig. 18 is a block diagram showing a configuration of a business proprietor terminal 510, which is a modified example of the business proprietor terminal 500 and enables the above-described process. The business proprietor terminal 510 includes an operation unit 511, a display unit 512, a determination unit 513, a communication unit 514, and an authentication unit 515.

The authentication unit 515 performs, for example, at least one of: biometric authentication such as face authentication, iris authentication, and fingerprint authentication; authentication by a code such as a two-dimensional code; individual authentication by a My Number; and electric authentication by eKYC (electronic Know Your Customer) in which individual verification information and face information are used in combination. As a result, the authentication unit 515 determines whether or not the individual to be authenticated corresponds to an individual who is registered as a person or the like permitted by the authentication unit 515. The authentication unit 515 functions, for example, as a photograph comparison unit.

Note that when the authentication unit 515 performs face authentication or eKYC authentication, the face image of the user used by the authentication unit 515 may be acquired by having the personal terminal 200 transmit a face image taken by the image-pickup unit 204 of the personal terminal 200 to the business proprietor terminal 510.

When the individual to be authenticated is not an individual who is registered as a person or the like permitted by the authentication unit 515, the determination unit 513 determines that the user should not be permitted to come to the office or the like, or to provide a service thereto.

When the individual to be authenticated is an individual who is registered as a person or the like permitted by the authentication unit 515, the communication unit 514 of the business proprietor terminal 510 transmits, as the information request F1, the authenticated individual identification information and an acquisition request for infectious-disease risk level information for that individual to the immunity passport base system 600. After receiving the information request F1, the immunity passport base system 600 performs secret calculation and transmits, as the information I2, the data of the infectious-disease risk level information, the hash value information of the immunity certificate of the individual, and the data of the digital signature to the business proprietor terminal 510.

The determination unit 513 of the business proprietor terminal 510 refers to the hash value and thereby makes a determination as to the tampering of the acquired data. Further, the determination unit 513 refers to the digital signature and thereby determines the genuineness of the acquired data. In this way, the business proprietor terminal 510 determines whether or not the infectious-disease risk level information of the user is valid.

Further, the determination unit 513 may verify the contents of the infectious-disease risk level information, and permits the user to come to the office or the like, or to provide a service thereto when the infectious disease risk is lower than or equal to a predetermined threshold. Details of the secret calculation and determination described above are the same as those described in the third example embodiment, and hence descriptions thereof are omitted. Through the above-described processes, the business proprietor terminal 510 can comprehensively determine the reliability of the individual based on the reliability of the infectious-disease risk level information and the result of the authentication by the authentication unit 515. Note that the order of determination processes performed by the business proprietor terminal 510 is not limited to the above-described order.

As described above, by providing the system on the business proprietor side with the configuration for individual authentication, even when a user who has a business purpose cannot present his/her infectious-disease risk level information, the information thereof can be obtained on the business proprietor side. Further, since the data of the immunity certificate itself is not presented to the business proprietor side, i.e., only the infectious-disease risk level information, which is the result of secret calculation, is presented to the business proprietor side, it is possible to take privacy into consideration and effectively use the immunity certificate for economic activities at the same time.

Although the above-described example embodiments have been described on the assumption that this disclosure is implemented as a hardware configuration, this disclosure is not limited to the hardware configuration. In this disclosure, the processes performed in the above-described example embodiments (e.g., the processes shown in the flowcharts shown in Figs. 14, 15 and 16) can also be carried out by having a processor execute a program.

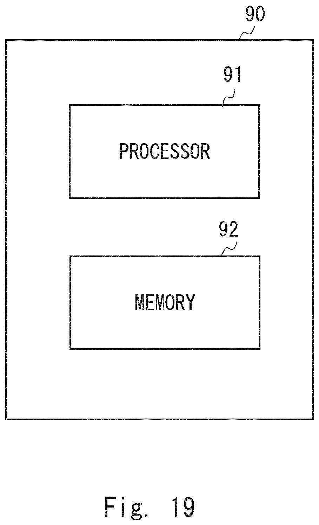

Fig. 19 is a block diagram showing an example of a hardware configuration of a computer in which the processes performed by the image-pickup apparatus according to each of the above-described example embodiments is executed. As shown in Fig. 19, this information processing apparatus 90 includes a processor 91 and a memory 92.

The processor 91 performs processes performed by the apparatuses described in the above-described example embodiments by loading software (a computer program) from the memory 92 and executing the loaded software. Note that, as the processor 91, one of a CPU (Central Processing Unit), an MPU (Micro Processing Unit), an FPGA (Field-Programmable Gate Array), a DSP (Demand-Side Platform), and an ASIC (Application Specific Integrated Circuit) may be used, or a plurality of processors or the like may be used in parallel.

The memory 92 is composed of a combination of a volatile memory and a nonvolatile memory. The memory 92 may include a storage remotely located from the processor 91. In this case, the processor 91 may access the memory 92 through an I/O (Input/Output) interface (not shown).

In the example shown in Fig. 19, the memory 92 is used to store a group of software modules. The processor 91 can perform the processes described in the above-described example embodiments by loading the group of software modules from the memory 92 and executing the loaded software modules.

As described above, one or a plurality of processors included in the image-pickup apparatus in the above-described example embodiments executes one or a plurality of programs including a group of instructions for causing a computer to perform the algorithm explained above with reference to the drawings. Through these processes, the processed described in each of the example embodiments can be implemented.

The program can be stored and supplied to a computer using various types of non-transitory computer readable media. The non-transitory computer readable media include various types of tangible storage media. Examples of non-transitory computer readable media include magnetic recording media (e.g., flexible disks, magnetic tapes, hard disk drives), magneto-optical recording media (e.g., magneto-optical disks), CD-ROM (Read Only Memory), CD-R, CD-R/W, and semiconductor memories (e.g., mask ROM, PROM (Programmable ROM), EPROM (Erasable PROM), flash ROM, and RAM (random access memory)). Further, the program may be supplied to the computer by various types of transitory computer readable media. Examples of transitory computer readable media include electrical signals, optical signals, and electromagnetic waves. The transitory computer readable medium can supply the program to the computer through a wired communication channel such as a wire and optical fiber, or through a wireless communication channel.

The whole or part of the example embodiments disclosed above can be described as, but not limited to, the following supplementary notes.

### (Supplementary note 1)

An information system comprising:
storage means for storing an immunity certificate;
update information storage means for storing, when the immunity certificate stored in the storage means is updated, update information of the immunity certificate as a hash value related to the immunity certificate; and
output means for outputting the update information stored in the update information storage means.

### (Supplementary note 2)

The information system described in Supplementary note 1, wherein the update information storage means stores a hash value related to the immunity certificate by incorporating the hash value into a transaction of a blockchain.

### (Supplementary note 3)

The information system described in Supplementary note 1 or 2, wherein the storage means stores the immunity certificate in a format for secret calculation.

### (Supplementary note 4)

The information system described in Supplementary note 3, further comprising secret calculation means for performing the secret calculation on the immunity certificate stored in the storage means, wherein
the output means outputs a result of the calculation performed by the secret calculation means and the update information.

### (Supplementary note 5)

The information system described in any one of Supplementary notes 1 to 4, wherein
the immunity certificate contains a digital signature,
the update information storage means stores the digital signature by incorporating the digital signature into the update information, and
the output means outputs the update information containing the digital signature.

### (Supplementary note 6)

The information system described in any one of Supplementary notes 1 to 5, further comprising receiving means for receiving update data of the immunity certificate from another terminal, wherein
when the information system receives the update data, the information system transmits information for notifying the other terminal of a caution about transmission of the update data.

### (Supplementary note 7)

An information system comprising:
storage means for storing an immunity certificate in a format for secret calculation;
secret calculation means for performing the secret calculation on the immunity certificate stored in the storage means; and
output means for outputting a result of the secret calculation performed by the secret calculation means.

### (Supplementary note 8)

The information system described in Supplementary note 7, further comprising update information storage means for storing, when the immunity certificate stored in the storage means is updated, update information of the immunity certificate as a hash value related to the immunity certificate, wherein
the output means outputs the update information stored in the update information storage means.

### (Supplementary note 9)

The information system described in Supplementary note 7 or 8, further comprising acquisition means for acquiring at least one of an algorithm and a parameter used for the secret calculation, wherein
when the acquisition means acquires the algorithm or the parameter, the secret calculation means updates the previous algorithm or the previous parameter used for the secret calculation with one acquired by the acquisition means.

### (Supplementary note 10)

The information system described in any one of Supplementary notes 7 to 9, wherein
the immunity certificate contains a movement history of an individual, and
the secret calculation means performs the secret calculation by using movement histories of individuals, contained in a plurality of immunity certificates, and thereby acquires a contact history between individuals as the result of the secret calculation.

### (Supplementary note 11)

The information system described in Supplementary note 10, wherein
the immunity certificate further contains infected-person information indicating whether or not an individual is infected with a specific infectious disease, and
the secret calculation means acquires, by performing the secret calculation using the infected-person information, a history indicating whether or not an individual has come into contact with an infected person as a contact history between individuals.

### (Supplementary note 12)

The information system described in any one of Supplementary notes 7 to 11, wherein the secret calculation means calculates, as the result of the secret calculation, a risk of an individual of infection of a specific infectious disease by using at least one of an algorithm and a parameter.

### (Supplementary note 13)

An information terminal comprising:
acquisition means for acquiring immunity certificate information;
sharing means for secretly sharing the immunity certificate information acquired by the acquisition means into a plurality of information pieces; and
transmitting means for transmitting the plurality of information pieces generated by the sharing means to another apparatus.

### (Supplementary note 14)

The information terminal described in Supplementary note 13, wherein the acquisition means is location information acquisition means for detecting location information of an individual through wireless communication, and thereby acquiring a location history.

### (Supplementary note 15)

The information terminal described in Supplementary note 13, wherein the acquisition means acquires biometric information of an individual as the immunity certificate information.

### (Supplementary note 16)

The information terminal described in any one of Supplementary notes 13 to 15, wherein the information terminal performs a pseudonymization process on the plurality of information pieces which the transmitting means transmits.

### (Supplementary note 17)

The information terminal described in any one of Supplementary notes 13 to 16, further comprising:
storage means for storing at least one of the immunity certificate information or information related to the immunity certificate information; and
display means for displaying at least one of the immunity certificate information or the information related to the immunity certificate information stored in the storage means.

### (Supplementary note 18)

An information terminal comprising:
acquisition means for acquiring information based on an immunity certificate of an individual;
receiving means for receiving a hash value from another apparatus as information indicating genuineness of the immunity certificate; and
determination means for determining, based on the hash value acquired by the receiving means, reliability of the information based on the immunity certificate.

### (Supplementary note 19)

The information terminal described in Supplementary note 18, wherein
the acquisition means further acquires issuer information of the immunity certificate,
the receiving means receives genuine issuer information from an issuer information management server, and
the determination means determines, based on the issuer information and the genuine issuer information acquired by the receiving means, reliability of the information based on the immunity certificate.

### (Supplementary note 20)

The information terminal described in Supplementary note 18 or 19, wherein the acquisition means is photographing means for acquiring the information based on the immunity certificate through photographing.

### (Supplementary note 21)

The information terminal described in any one of Supplementary notes 18 to 20, further comprising authentication means for authenticating an individual by acquiring personal information, wherein
the determination means determines reliability of an individual based on reliability of the information based on the immunity certificate and the result of the authentication by the authentication means.

### (Supplementary note 22)

An immunity certificate management system comprising:
an information system; and
an information terminal configured to communicate with the information system, wherein
the information system comprises:
   storage means for storing an immunity certificate;
   update information storage means for storing, when the immunity certificate stored in the storage means is updated, update information of the immunity certificate as a hash value related to the immunity certificate; and
   transmitting means for transmitting the update information stored in the update information storage means to the information terminal, and
   the information terminal comprises receiving means for receiving the transmitted update information.

### (Supplementary note 23)

The immunity certificate management system described in Supplementary note 22, wherein the storage means stores the immunity certificate in a format for secret calculation.

### (Supplementary note 24)

An immunity certificate management system comprising:
an information system; and
an information terminal configured to communicate with the information system, wherein
the information system comprises:
   storage means for storing an immunity certificate in a format for secret calculation;
   secret calculation means for performing the secret calculation on the immunity certificate stored in the storage means; and
   transmitting means for transmitting a result of the secret calculation performed by the secret calculation means to the information terminal, and
   the information terminal comprises receiving means for receiving the result of the secret calculation.

### (Supplementary note 25)

The immunity certificate management system described in Supplementary note 24, wherein
the information system further comprises update information storage means for storing, when the immunity certificate stored in the storage means is updated, update information of the immunity certificate as a hash value related to the immunity certificate, and
the output means outputs the update information stored in the update information storage means to the information terminal.

### (Supplementary note 26)

An information processing method comprising:
a step of storing an immunity certificate;
a step of storing, when the stored immunity certificate is updated, update information of the immunity certificate as a hash value related to the immunity certificate; and
a step of outputting the stored update information.

### (Supplementary note 27)

An information processing method comprising:
a step of storing an immunity certificate in a format for secret calculation;
a step of performing the secret calculation on the stored immunity certificate; and
a step of outputting a result of the secret calculation.

### (Supplementary note 28)

An information processing method comprising:
a step of acquiring immunity certificate information;
a step of secretly sharing the acquired immunity certificate information into a plurality of information pieces; and
a step of transmitting the secrecy-shared plurality of information pieces to another apparatus.

### (Supplementary note 29)

An information processing method comprising:
a step of acquiring information based on an immunity certificate of an individual;
a step of communicating with another apparatus and thereby acquiring a hash value from the other apparatus as information indicating genuineness of the immunity certificate; and
a step of determining, based on the acquired hash value, reliability of the information based on the immunity certificate.

### (Supplementary note 30)

A non-transitory computer readable medium storing a program for causing a computer to perform:
a step of storing an immunity certificate;
a step of storing, when the stored immunity certificate is updated, update information of the immunity certificate as a hash value related to the immunity certificate; and
a step of outputting the stored update information.

### (Supplementary note 31)

A non-transitory computer readable medium storing a program for causing a computer to perform:
a step of storing an immunity certificate in a format for secret calculation;
a step of performing the secret calculation on the stored immunity certificate; and
a step of outputting a result of the secret calculation.

### (Supplementary note 32)

A non-transitory computer readable medium storing a program for causing a computer to perform:
a step of acquiring immunity certificate information;
a step of secretly sharing the acquired immunity certificate information into a plurality of information pieces; and
a step of transmitting the secrecy-shared plurality of information pieces to another apparatus.

### (Supplementary note 33)

A non-transitory computer readable medium storing a program for causing a computer to perform:
a step of acquiring information based on an immunity certificate of an individual;
a step of communicating with another apparatus and thereby acquiring a hash value from the other apparatus as information indicating genuineness of the immunity certificate; and
a step of determining, based on the acquired hash value, reliability of the information based on the immunity certificate.

Although the present disclosure has been described above with reference to example embodiments, the present disclosure is not limited to the above-described example embodiments. Various modifications that can be understood by those skilled in the art can be made to the configuration and details of the present disclosure within the scope of the disclosure.

### Reference Signs List

- 1: IMMUNITY CERTIFICATE MANAGEMENT SYSTEM
- 10: INFORMATION TERMINAL
- 11: ACQUISITION UNIT
- 12: SHARING UNIT
- 13: TRANSMITTING UNIT
- 20: INFORMATION TERMINAL
- 21: ACQUISITION UNIT
- 22: RECEIVING UNIT
- 23: DETERMINATION UNIT
- 30, 40: INFORMATION SYSTEM
- 31, 41: STORAGE UNIT
- 32: UPDATE INFORMATION STORAGE UNIT
- 42: SECRET CALCULATION UNIT
- 33, 43: OUTPUT UNIT
- 100: IMMUNITY PASSPORT SYSTEM
- 200, 210: PERSONAL TERMINAL
- 201,211: OPERATION UNIT
- 202, 212: LOCATION INFORMATION ACQUISITION UNIT
- 203, 213: DISPLAY UNIT
- 204: IMAGE-PICKUP UNIT
- 214: BIOMETRIC INFORMATION ACQUISITION UNIT
- 205, 215: STORAGE UNIT
- 206, 216: SECRET SHARING UNIT
- 207, 217: DETERMINATION UNIT
- 208, 218: COMMUNICATION UNIT
- 300: MEDICAL INSTITUTION TERMINAL
- 301: OPERATION UNIT
- 302: DISPLAY UNIT
- 303: COMMUNICATION UNIT
- 400: ADMINISTRATIVE INSTITUTION TERMINAL
- 401: OPERATION UNIT
- 402: DISPLAY UNIT
- 403: TARGET SPECIFYING UNIT
- 404: COMMUNICATION UNIT
- 500: BUSINESS PROPRIETOR TERMINAL
- 501: OPERATION UNIT
- 502: DISPLAY UNIT
- 503: DETERMINATION UNIT
- 504: COMMUNICATION UNIT
- 510: BUSINESS PROPRIETOR TERMINAL
- 511: OPERATION UNIT
- 512: DISPLAY UNIT
- 513: DETERMINATION UNIT
- 514: COMMUNICATION UNIT
- 515: AUTHENTICATION UNIT
- 600: IMMUNE PASSPORT BASE SYSTEM
- 601: STORAGE UNIT
- 602: BLOCKCHAIN STORAGE UNIT
- 603: UPDATE UNIT
- 604: SECRET CALCULATION UNIT
- 605: EXTRACTION UNIT
- 606: COMMUNICATION UNIT

## Claims

1. An information system comprising:
storage means for storing an immunity certificate;
update information storage means for storing, when the immunity certificate stored in the storage means is updated, update information of the immunity certificate as a hash value related to the immunity certificate; and
output means for outputting the update information stored in the update information storage means.

2. The information system according to Claim 1, wherein the update information storage means stores a hash value related to the immunity certificate by incorporating the hash value into a transaction of a blockchain.

3. The information system according to Claim 1 or 2, wherein the storage means stores the immunity certificate in a format for secret calculation.

4. The information system according to Claim 3, further comprising secret calculation means for performing the secret calculation on the immunity certificate stored in the storage means, wherein
the output means outputs a result of the calculation performed by the secret calculation means and the update information.

5. The information system according to any one of Claims 1 to 4, wherein
the immunity certificate contains a digital signature,
the update information storage means stores the digital signature by incorporating the digital signature into the update information, and
the output means outputs the update information containing the digital signature.

6. The information system according to any one of Claims 1 to 5, further comprising receiving means for receiving update data of the immunity certificate from another terminal, wherein
when the information system receives the update data, the information system transmits information for notifying the other terminal of a caution about transmission of the update data.

7. An information system comprising:
storage means for storing an immunity certificate in a format for secret calculation;
secret calculation means for performing the secret calculation on the immunity certificate stored in the storage means; and
output means for outputting a result of the secret calculation performed by the secret calculation means.

8. The information system according to Claim 7, further comprising update information storage means for storing, when the immunity certificate stored in the storage means is updated, update information of the immunity certificate as a hash value related to the immunity certificate, wherein
the output means outputs the update information stored in the update information storage means.

9. The information system according to Claim 7 or 8, further comprising acquisition means for acquiring at least one of an algorithm and a parameter used for the secret calculation, wherein
when the acquisition means acquires the algorithm or the parameter, the secret calculation means updates the previous algorithm or the previous parameter used for the secret calculation with one acquired by the acquisition means.

10. The information system according to any one of Claims 7 to 9, wherein
the immunity certificate contains a movement history of an individual, and
the secret calculation means performs the secret calculation by using movement histories of individuals, contained in a plurality of immunity certificates, and thereby acquires a contact history between individuals as the result of the secret calculation.

11. The information system according to Claim 10, wherein
the immunity certificate further contains infected-person information indicating whether or not an individual is infected with a specific infectious disease, and
the secret calculation means acquires, by performing the secret calculation using the infected-person information, a history indicating whether or not an individual has come into contact with an infected person as a contact history between individuals.

12. The information system according to any one of Claims 7 to 11,
wherein the secret calculation means calculates, as the result of the secret calculation, a risk of an individual of infection of a specific infectious disease by using at least one of an algorithm and a parameter.

13. An information terminal comprising:
acquisition means for acquiring immunity certificate information;
sharing means for secretly sharing the immunity certificate information acquired by the acquisition means into a plurality of information pieces; and
transmitting means for transmitting the plurality of information pieces generated by the sharing means to another apparatus.

14. The information terminal according to Claim 13, wherein the acquisition means is location information acquisition means for detecting location information of an individual through wireless communication, and thereby acquiring a location history.

15. The information terminal according to Claim 13, wherein the acquisition means acquires biometric information of an individual as the immunity certificate information.

16. The information terminal according to any one of Claims 13 to 15, wherein the information terminal performs a pseudonymization process on the plurality of information pieces which the transmitting means transmits.

17. The information terminal according to any one of Claims 13 to 16, further comprising:
storage means for storing at least one of the immunity certificate information or information related to the immunity certificate information; and
display means for displaying at least one of the immunity certificate information or the information related to the immunity certificate information stored in the storage means.

18. An information terminal comprising:
acquisition means for acquiring information based on an immunity certificate of an individual;
receiving means for receiving a hash value from another apparatus as information indicating genuineness of the immunity certificate; and
determination means for determining, based on the hash value acquired by the receiving means, reliability of the information based on the immunity certificate.

19. The information terminal according to Claim 18, wherein
the acquisition means further acquires issuer information of the immunity certificate,
the receiving means receives genuine issuer information from an issuer information management server, and
the determination means determines, based on the issuer information and the genuine issuer information acquired by the receiving means, reliability of the information based on the immunity certificate.

20. The information terminal according to Claim 18 or 19, wherein the acquisition means is photographing means for acquiring the information based on the immunity certificate through photographing.

21. The information terminal according to any one of Claims 18 to 20, further comprising authentication means for authenticating an individual by acquiring personal information, wherein
the determination means determines reliability of an individual based on reliability of the information based on the immunity certificate and the result of the authentication by the authentication means.

22. An immunity certificate management system comprising:
an information system; and
an information terminal configured to communicate with the information system, wherein
the information system comprises:
storage means for storing an immunity certificate;
update information storage means for storing, when the immunity certificate stored in the storage means is updated, update information of the immunity certificate as a hash value related to the immunity certificate; and
transmitting means for transmitting the update information stored in the update information storage means to the information terminal, and
the information terminal comprises receiving means for receiving the transmitted update information.

23. The immunity certificate management system according to Claim 22, wherein the storage means stores the immunity certificate in a format for secret calculation.

24. An immunity certificate management system comprising:
an information system; and
an information terminal configured to communicate with the information system, wherein
the information system comprises:
storage means for storing an immunity certificate in a format for secret calculation;
secret calculation means for performing the secret calculation on the immunity certificate stored in the storage means; and
transmitting means for transmitting a result of the secret calculation performed by the secret calculation means to the information terminal, and
the information terminal comprises receiving means for receiving the result of the secret calculation.

25. The immunity certificate management system according to Claim 24, wherein
the information system further comprises update information storage means for storing, when the immunity certificate stored in the storage means is updated, update information of the immunity certificate as a hash value related to the immunity certificate, and
the transmitting means transmits the update information stored in the update information storage means to the information terminal.

26. An information processing method comprising:
a step of storing an immunity certificate;
a step of storing, when the stored immunity certificate is updated, update information of the immunity certificate as a hash value related to the immunity certificate; and
a step of outputting the stored update information.

27. An information processing method comprising:
a step of storing an immunity certificate in a format for secret calculation;
a step of performing the secret calculation on the stored immunity certificate; and
a step of outputting a result of the secret calculation.

28. An information processing method comprising:
a step of acquiring immunity certificate information;
a step of secretly sharing the acquired immunity certificate information into a plurality of information pieces; and
a step of transmitting the secrecy-shared plurality of information pieces to another apparatus.

29. An information processing method comprising:
a step of acquiring information based on an immunity certificate of an individual;
a step of communicating with another apparatus and thereby acquiring a hash value from the other apparatus as information indicating genuineness of the immunity certificate; and
a step of determining, based on the acquired hash value, reliability of the information based on the immunity certificate.

30. A non-transitory computer readable medium storing a program for causing a computer to perform:
a step of storing an immunity certificate;
a step of storing, when the stored immunity certificate is updated, update information of the immunity certificate as a hash value related to the immunity certificate; and
a step of outputting the stored update information.

31. A non-transitory computer readable medium storing a program for causing a computer to perform:
a step of storing an immunity certificate in a format for secret calculation;
a step of performing the secret calculation on the stored immunity certificate; and
a step of outputting a result of the secret calculation.

32. A non-transitory computer readable medium storing a program for causing a computer to perform:
a step of acquiring immunity certificate information;
a step of secretly sharing the acquired immunity certificate information into a plurality of information pieces; and
a step of transmitting the secrecy-shared plurality of information pieces to another apparatus.

33. A non-transitory computer readable medium storing a program for causing a computer to perform:
a step of acquiring information based on an immunity certificate of an individual;
a step of communicating with another apparatus and thereby acquiring a hash value from the other apparatus as information indicating genuineness of the immunity certificate; and
a step of determining, based on the acquired hash value, reliability of the information based on the immunity certificate.
